# EUROPEAN PATENT SPECIFICATION

(11) **EP 4 444 209 B1**
(45) Date of publication and mention of the grant of the patent: **18.03.2026**
(21) Application number: 22823021.5
(22) Date of filing: 29.11.2022
(51) Int. Cl.: A61B 18/20

(54) **APPARATUS FOR CONTROLLING A TREATMENT DEVICE AND SYSTEM COMPRISING A TREATMENT DEVICE**
LICHTBEHANDLUNGSVORRICHTUNG ZUR HAUTBEHANDLUNG
DISPOSITIF DE TRAITEMENT POUR LE TRAITEMENT DE LA PEAU PAR LA LUMIERE

(30) Priority: 09.12.2021 EP 21213384
(43) Date of publication of application: 16.10.2024
(60) Divisional application: 25222086.8
(73) Proprietor: Koninklijke Philips N.V., 5656 AG Eindhoven (NL)
(72) Inventor: VERKRUIJSSE, Willem, 5656 AG Eindhoven (NL); BOURQUIN, Yannyk, Parulian, Julian, 5656 AG Eindhoven (NL); THUMMA, Kiran, Kumar, 5656 AG Eindhoven (NL); VARGHESE, Babu, 5656 AG Eindhoven (NL); PALERO, Jonathan, Alambra, 5656AG Eindhoven (NL)
(74) Representative: Philips Intellectual Property & Standards
(86) International application number: PCT/EP2022/083643
(87) International publication number: WO 2023/104587

(56) References cited:
- EP-A1- 3 842 002
- EP-A1- 3 915 502
- US-A1- 2021 322 098

## Description

### FIELD OF THE INVENTION

The present invention relates to an apparatus configured to control a treatment device configured to perform a treatment operation on a body part of a subject. The present invention relates further to a computer-implemented non-therapeutic photo-epilation method of controlling such a treatment device, and to a system comprising such an apparatus and such a treatment device.

### BACKGROUND OF THE INVENTION

Techniques for removal of unwanted hairs include shaving, electrolysis, plucking, laser and light therapies (known as photo-epilation) and injection of therapeutic anti-androgens. Light-based technologies are also used in other types of dermatological treatments, including hair growth reduction and treating acne.

Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. Home-use photo-epilation devices, for example the Philips Lumea device, use intense pulsed light (IPL) from high intensity light sources, e.g. Xenon flash lamps that produce high output bursts of broad spectrum light.

A photo-epilation treatment is characterized by the user of the photo-epilation device treating relatively small areas of the skin for the purpose of hair removal. The photo-epilation treatment uses intense light to heat melanin in hair and hair roots, which puts the hair follicles into a resting phase, preventing hair re-growth. For effective use of this technology for hair removal, the user must treat the skin completely without leaving any gaps. Since this effect is only of limited duration, treatment has to be repeated on a regular basis: typically once every 4 to 8 weeks in the maintenance phase after an initial period of about two months in which treatment is performed once every two weeks.

Photo-epilation is currently done by the user by careful `step and flash' mode or automated 'slide and flash' mode. 'Step and flash' mode for the treatment on the skin requires focus for a careful placement of the device for the next treatment spot to avoid missed spots. The user is not given any feedback for the placement of the device after the previous treated area. Concentration and quite precise manual coordination are required in the placement of the device for the next spot. This can be a rather slow process which leads to low efficacy and/or discomfort as the user perceives pain sensation as he anticipates the arrival of the pulse. Furthermore, the user also feels discomfort to step and flash as he/she needs to focus on the treatment. On the other hand, 'slide and flash' mode has the advantage of automatically flashing at a fixed time (2-3.5s), however there is burden to the user to move the device in a controlled manner such that it is not too slow as to overtreat areas, and not too fast as to miss spots, and this speed should be maintained during the entire treatment.

WO 2021/130052 A1 discloses an apparatus for use with a treatment device for providing feedback to a user on a treatment operation performed on a body part of a subject. The treatment device is configured to apply light pulses to skin of the body part to perform the treatment operation, wherein a light pulse applied to the skin treats an area of the skin. The apparatus comprises a processing unit configured to receive a first measurement signal from a first sensor, the first measurement signal comprising information about positions and/or movements of the treatment device over time. For a light pulse previously applied by the treatment device to the body part during the treatment operation, the first measurement signal is processed to estimate a previous treatment position as a position of the treatment device relative to the body part when the light pulse was generated. For the previously applied light pulse and based on the estimated previous treatment position, a previous treatment area is estimated for the light pulse corresponding to the area of skin of the body part that the light pulse was applied to when the treatment device was at the previous treatment position. The first measurement signal is processed to estimate a current position of the treatment device relative to the body part. Based on the estimated current position of the treatment device, a current treatment area corresponding to an area of skin that the treatment device would apply a light pulse to while in the current position is estimated. A feedback control signal for a feedback unit is generated, wherein the feedback control signal is configured to cause the feedback unit to generate feedback indicating whether the current treatment area corresponds, or substantially corresponds, to a previous treatment area. Furthermore, in the context of light treatment devices for skin treatment, the technical teaching provided by document US2021/0322098 A1 is acknowledged.

### SUMMARY OF THE INVENTION

It is an object of the present invention to overcome the disadvantages of the different modes of operation of a treatment device, in particular to adapt the operation of the treatment device to various speeds of movement of the treatment device over the skin, while obtaining optimal treatment coverage (i.e. avoid overtreatment and avoid missed areas).

The invention is defined in the appended set of claims. In a first aspect of the present invention an apparatus configured to control a treatment device is presented. The treatment device is configured to perform a treatment operation on a body part of a subject, wherein the treatment device is configured to apply light pulses to skin of the body part to perform the treatment operation, wherein a light pulse applied to the skin treats a treatment area of the skin, the treatment area having a known or estimated width in a movement direction of the treatment device. The apparatus comprises a processing unit configured to:
- estimate a position of a current treatment area corresponding to an area of skin that the treatment device would apply a light pulse to while at a current treatment position;
- determine a distance, in the movement direction of the treatment device, of the current treatment area from a previous treatment area corresponding to an area of skin that the treatment device applied a previous light pulse to while at a previous treatment position;
- determine if the distance is zero;
- generate a pulse control signal for controlling the treatment device to generate and apply a light pulse to the current treatment area of the skin if the distance has been determined to be zero;
- determine if the distance is an integer multiple of the width of the treatment area; and
- generate a pulse control signal for controlling the treatment device to generate and apply a light pulse to the current treatment area of the skin if the distance has been determined to be said integer multiple of the width of the treatment area.

In a second aspect of the present invention another apparatus configured to control a treatment device is presented. The treatment device is configured to perform a treatment operation on a body part of a subject, wherein the treatment device is configured to apply light pulses to skin of the body part to perform the treatment operation, wherein a light pulse applied to the skin treats a treatment area of the skin, the treatment area having a known or estimated width in a movement direction of the treatment device. The apparatus comprises a processing unit configured to:
- determine a distance, in the movement direction of the treatment device, that the treatment device has moved;
- generate, based on a pre-set speed of the treatment device, a pulse control signal for controlling the treatment device to generate and apply a light pulse if the determined distance corresponds to the width of the treatment area;
- set an integer multiple of the width of the treatment area based on the pre-set speed of the treatment device, wherein said integer multiple is at least two; and
- generate the pulse control signal for controlling the treatment device to generate and apply a light pulse if the determined distance corresponds to the set integer multiple of the width of the treatment area.

In a further aspect of the present invention a system is presented comprising:
a treatment device comprising one or more light sources for generating light pulses for application to skin of the body to perform a treatment operation;
a sensor for outputting a first measurement signal comprising information about positions and/or movements of the treatment device over time; and
an apparatus as disclosed herein.

In yet further aspects of the present invention, there are provided a corresponding non-therapeutic photo-epilation method, a computer program which comprises program code means for causing a computer to perform the steps of the methods disclosed herein when said computer program is carried out on a computer, as well as a non-transitory computer-readable recording medium that stores therein a computer program product, which, when executed by a processor, causes the methods disclosed herein to be performed.

Preferred embodiments of the invention are defined in the dependent claims. It shall be understood that the claimed methods, system, computer program and medium have similar and/or identical preferred embodiments as the claimed apparatus, in particular as defined in the dependent claims and as disclosed herein.

Applying the right speed when using the device in step and flash mode is difficult. When moving too slow, the treatment takes longer and there is a risk of overlapping. When moving too fast, there is a risk of missing parts of the skin that will not be treated. Furthermore, step and flash mode may take too long time and cause discomfort to the user as the user needs to focus on placing the device right next to the treated area. To counter such problems the present invention enables the treatment device to be moved (i.e. to glide) like in a shaving motion, with variable speed (i.e., there is no need of a constant speed or a constant movement distance in a step and flash mode), and treat the skin without missing spots and without overtreated spots. This helps the user to make the treatment more easy and comfortable. Furthermore, the user needs to focus only on moving the treatment device and does not need to care about the next pulse emission (also called flash herein) for skin treatment. A gliding motion is easier to do for the user but it may also reduce the perceived pain sensation since there is less concentration on the flash. Overall, the present invention enables an optimal treatment and gives the user the flexibility to use the treatment device at any desired speed.

According to the present invention the treatment area can be tracked and the flashing can be automated to avoid missed spots and overtreatment in a treatment strip (comprising multiple treatments areas). Further, the overall power consumption of the device can be improved by flashing only when required (i.e., by flashing in a deterministic manner). Thus, large areas (like legs, back, chest) can be treated in an efficient way by treating one or more treatment strips in a way that avoids any missed spots and, as optionally provided in an embodiment, placing these strips next to each other.

According to the first aspect of the present invention, this is achieved by controlling the flashing such that the size of gaps between treated treatment areas, i.e., the distance of a current treatment area from a previous treatment area in the movement direction of the treatment device, is either zero (without overlap between two adjacent treatment areas) or an integer multiple of the width of the treatment area. **In** other words, it is controlled, during movement of the treatment of the treatment device over the skin, when to flash and when not to flash. Thus, untreated treatment areas that have not been flashed in a first stroke (i.e. a first directional movement of the treatment device over a certain portion of skin covering a number of treatment areas) are then flashed in a second (and, optionally, even further) stroke(s) over the same portion of skin, preferably in alternating opposite direction(s). The width of the treatment area is (at least roughly) known or estimated due to the fixed size of the aperture for the emitted light in the treatment device. Even if the speed of movement changes during a stroke or from one stroke to another stroke, it can thus be ensured that the desired portion of skin is substantially homogeneously treated without having (substantial or any) overlaps and without having (substantial or any) gaps between neighboring treatment areas.

According to the second aspect of the present invention, the desired effect is achieved by setting the integer multiple of the width of the treatment area in advance based on a pre-set speed (e.g. low, medium, high) of the treatment device and to control the flashing based on the distance that the treatment device has moved during treatment, such that flashing occurs when the determined distance corresponds to the set integer multiple of the width of the treatment area. Like according to the first aspect, it is controlled when to flash and when not to flash so that the desired arrangement of the treated and untreated treatment areas, as described above, is resulting. A certain portion of the skin can thus be treated without overtreatment or no treatment at all of any spots.

According to an embodiment the processing unit is configured to determine the integer multiple based on an initial movement speed of the treatment device, in particular a measured or pre-set movement speed of the treatment device and the known or estimated width of the treatment area. The initial movement speed may e.g. be selected by a user from a menu of available speeds (such as low, medium, high), in which the user intends to move the treatment device over the skin. The speed can be used to make an initial determination of the integer multiple of the width of the treatment area and thus to determine initially, at which positions (provided the treatment device is moved according to the initial movement speed) pulses shall be emitted for treatment.

Further, the processing unit may be configured to determine a number of strokes needed to treat a first portion of skin. For instance, if a high movement speed is used, the gaps between treated treatment areas in a first stroke may be larger since there may be sufficient time needed for powering in between two subsequently emitted pulses. Photo-epilation devices also feature different treatment head attachments of various aperture sizes. This together with the total area of a specific treatment region, e.g. upper lip, legs, armpit, may be used as well to determine the number of strokes and/or the size and/or number of gaps between treated treatment areas in a first stroke. Thus, depending of the speed of movement it may be computed in advance, how many strokes may be needed to completely cover a certain portion of skin.

In another embodiment the processing unit is further configured to adjust the integer multiple and/or the number of strokes upon determining that a current movement speed of the treatment device is different from the initial movement speed. Thus, preferably the movement speed and/or the distance moved during a certain time period is measured and used to re-calculate the initially determined integer multiple and/or number of strokes to ensure that the desired portion of skin is covered in the best way and/or in the minimum time.

The processing unit may further be configured to determine if the current treatment area overlaps with the previous treatment area, in particular by determining if a light pulse has been previously applied to the current treatment area, and generate the pulse control signal if the distance has been determined to be zero or an integer multiple of the width of the treatment area and if the current treatment area has been determined not to overlap with the previous treatment area. This additional check ensures avoiding overtreatment of skin areas.

The processing unit may further be configured to determine if the treatment device is ready to apply a light pulse, in particular if a light source of the treatment device has sufficient energy to apply a light pulse, and generate the pulse control signal if the distance has been determined to be zero or an integer multiple of the width of the treatment area and if the treatment device has been determined to be ready to apply a light pulse. This additional check contributes to minimizing the time needed for treatment of a certain portion of skin.

**In** another embodiment the processing unit is configured to generate a guidance signal for a feedback unit, wherein the guidance signal is configured to cause the feedback unit to generate guidance indicating into which direction and/or at which treatment area the treatment device shall be moved. This helps the user to correctly and fully cover a desired portion of skin.

The processing unit may hereby be configured to:
- determine if, after a first stroke of the treatment device over a first portion of the skin, there are untreated treatment areas between treated treatment areas; and
- generate the guidance signal for the feedback unit, wherein the guidance signal is configured to cause the feedback unit to generate guidance indicating:
- that the treatment device shall be moved in a second stroke over the same first portion of the skin but in the opposite direction if there are untreated treatment areas between treated treatment areas in the first portion of the skin, or
- that the treatment of the first portion of the skin is finished and/or that the treatment device shall be moved in a second stroke over a second portion of the skin different from the first portion if there are no untreated treatment areas between treated treatment areas in the first portion of the skin.

The guidance signal may e.g. be configured to cause the feedback unit to generate guidance indicating that the treatment device shall be moved in the second stroke over a second portion of the skin that is parallel and adjacent to the first portion of the skin if there are no untreated treatment areas between treated treatment areas in the first portion of the skin. A larger portion of skin may thus be treated in smaller portions that are arranged adjacent to each other and that are treated in a way like a lawn is mowed, wherein each portion may be treated by one or more strokes.

The processing unit may further be configured to compute a map indicating treated treatment areas to which a light pulse has been previously applied and untreated treatment areas to which a light pulse has not been previously applied and generate the guidance signal for the feedback unit based on the computed map. Optionally, the map may be visualized to the user so that the user can see which areas still have to be treated.

A possible way to determine the position of a treatment area is provided in an embodiment according to which the processing unit is configured to:
receive a first measurement signal from a sensor, the first measurement signal comprising information about positions and/or movements of the treatment device over time;
for a light pulse previously applied by the treatment device to the body part during the treatment operation, process the first measurement signal to estimate the previous treatment position as a position of the treatment device relative to the body part when the light pulse was generated;
for the previously applied light pulse and based on the estimated previous treatment position, estimate the position of the previous treatment area;
process the first measurement signal to estimate the current position (and/or orientation) of the treatment device relative to the body part; and
based on the estimated current position (and/or orientation) of the treatment device, estimate the position of the current treatment area.

The processing unit may further be configured to determine movement direction and/or speed and/or orientation of the treatment device, and generate the guidance signal for the feedback unit, wherein the guidance signal is configured to cause the feedback unit to generate guidance indicating into which direction and/or at which treatment area the treatment device shall be moved and/or if and how the speed of movement of the treatment device shall be changed. This supports the user in the correct movement of the treatment device.

The processing unit may optionally be configured to generate the guidance signal for controlling the feedback unit to generate visible and/or audible guidance, in particular guidance in the form of audible instructions and/or in the form of a graphical representation of instructions and/or the computed map and/or previous treatment areas and the current treatment area and/or a pattern or line. For instance, a visual guidance (lines, arrows, text, etc.) may be displayed on the skin. In an example a line may be displayed along which the treatment device shall be moved.

The guidance signal may e.g. be a display control signal. For the feedback unit different implementations are possible. For instance, the feedback unit may be a display screen and the display control signal includes a plurality of images or a video sequence of the body part on which the graphical representation is overlaid, or the feedback unit may be a projector and the display control signal is configured to cause the projector to project the graphical representation onto the body part of the subject, or the feedback unit may be configured to display the graphical representation as part of an augmented reality display of the body part.

In the apparatus of the second aspect, the processing unit may be configured to set a number of strokes needed for treating a first portion of skin based on the set integer multiple of the width of the treatment area. Thus, it may be computed in advance, how many strokes may be needed. The user may be informed in advance or during treatment of this number.

Further, in the apparatus of the second aspect, the processing unit may be configured to re-calculate the integer multiple of the width of the treatment area and/or the number of strokes if the speed of movement of the treatment device changes or is different from the pre-set speed. Thus, even if the user changes the speed of movement during treatment, the aims of avoiding overtreatment and of avoiding missed spots can be achieved.

### BRIEF DESCRIPTION OF THE DRAWINGS

These and other aspects of the invention will be apparent from and elucidated with reference to the embodiment(s) described hereinafter. In the following drawings
Fig. 1 shows an illustration of an exemplary embodiment of a treatment device with which the invention can be used;
Fig. 2 shows a schematic diagram of an exemplary embodiment of a system according to the present invention;
Fig. 3 shows an infra-red image of an arm after a number of light pulses have been applied;
Fig. 4 shows a schematic diagram of a typical treatment with a conventional treatment device;
Fig. 5 shows a flow chart of an exemplary embodiment of a method according to a first aspect of the present invention;
Fig. 6 shows a schematic diagram for illustrating the steps of the method of the present invention;
Fig. 7 shows diagrams illustrating the different strokes for the strip filling concept shown in Fig. 6;
Fig. 8 shows a diagram illustrating the filling of a strip when the user applies a slow movement speed during treatment;
Fig. 9 shows diagrams illustrating the filling of a strip when the user applies a moderate movement speed during treatment;
Fig. 10 shows diagrams illustrating the filling of a strip when the user applies a fast movement speed during treatment;
Fig. 11 shows diagrams illustrating the filling of a strip when the user applies a variable movement speed during treatment;
Fig. 12 shows a flow chart of an exemplary method according to the present invention for determining the current position of the treatment device;
Fig. 13 shows two images 66 of a body part 69 obtained by an imaging unit;
Fig. 14 shows a flow chart of an exemplary embodiment of a method according to a second aspect of the present invention; and
Fig. 15 shows a schematic diagram of another embodiment of a treatment device according to the present invention.

### DETAILED DESCRIPTION OF EMBODIMENTS

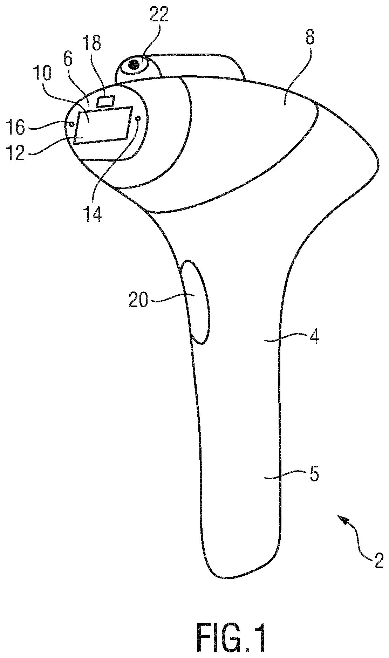

Fig. 1 is an illustration of an exemplary treatment device 2 that can be used to apply a light pulse to an area of skin. It will be appreciated that the treatment device 2 in Fig. 1 is merely presented as an example of a hand-held treatment device 2 that the invention can be used with, and the treatment device 2 is not limited to the form shown in Fig. 1 or to being a hand-held treatment device. The treatment device 2 is for use on a body of a subject (e.g. a person or an animal), and is to be held in one or both hands of a user during use. The treatment device 2 is to perform some treatment operation to the skin or body of the subject using one or more light pulses when the treatment device 2 is in contact with, or close to, a body part of the subject. The treatment operation can be removal of unwanted hairs by laser and/or light therapies (known as a photo-epilation treatment or Intense Pulsed Light, IPL, treatment). Alternatively the treatment operation can be laser and/or light therapies for reasons other than removal or preventing growth of hair, such as a dermatological (skin) treatment, treating acne, a phototherapy treatment for other types of skin condition, skin rejuvenation, skin tightening, or port-wine stain treatment.

As described herein, the treatment device 2 is operated or used by a 'user', and the treatment device 2 is used on a body of a 'subject'. In some cases the user and the subject is the same person, i.e. the treatment device 2 is held in a hand and used by a user on themselves (e.g. used on the skin on their leg). In other cases the user and the subject are different people, e.g. the treatment device 2 is held in a hand and used by a user on someone else. In either case, it is difficult for a user to achieve complete coverage of a body part and/or avoid over-treating certain areas of the body part since there are little or no user-perceptible changes to the skin on applying or shortly after applying a light pulse.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the treatment operation to be performed on the body or skin of the subject at the position that the head portion 6 is in contact with the body or skin.

The treatment device 2 is for performing a treatment operation using light pulses. Thus, in Fig. 1 the head portion 6 comprises an aperture 10 arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture 10 may be in the form of an opening at the head end 8 of the housing 4, or it may be in the form of a window (including a waveguide) that is transparent or semitransparent to the light pulses (i.e. the light pulses can pass through the window).

In the exemplary embodiment shown in Fig. 1, the aperture 10 has a generally rectangular shape, which results in a generally rectangular-shaped skin treatment region on the skin. It will be appreciated that the aperture 10 can have any other desired shape. For example the aperture 10 can be square, elliptical, circular, or any other polygonal shape.

Although not shown in Fig. 1, the treatment device 2 may have one or more removable attachments for the head portion 6 of the treatment device 2 that each includes an aperture 10. The attachments may be for use on different body parts, and have apertures 10 with different sizes. For example one attachment can be for use on a large body part such as the legs and therefore have a large aperture 10, whereas another attachment can be for use on the skin/hair above the upper lip and therefore have a small aperture 10.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any suitable type of light source, such as one or more light emitting diodes (LEDs), a (Xenon) flash lamp, a laser or lasers, etc. The light source(s) 12 can provide light pulses with spectral content in the 560-1200 nm range for a duration of around 2.5 ms, as these wavelengths heat melanin in the hair and hair root by absorption, which puts the hair follicles in a resting phase, preventing hair regrowth.

The one or more light sources 12 are configured to provide pulses of light. That is, the light source(s) 12 are configured to generate light at a high intensity for a short duration (e.g. less than 1 second). The intensity of the light pulse should be high enough to effect the treatment operation on the skin or body part adjacent the aperture 10.

The illustrated treatment device 2 may optionally include two skin contact sensors 14, 16 positioned on or in the head portion 6 that are used to determine whether the head portion 6 is in contact with the skin. The skin contact sensors 14, 16 measure a parameter that is indicative of whether the head portion 6 is in contact with skin, and generate respective measurement signals that comprise a time-series of measurements of the parameter. The measurement signals can be processed to determine if the head portion 6 is in contact with skin. Typically a skin contact sensor is used in a treatment device 2, particularly a photoepilator, to make sure that the treatment device 2 is correctly in contact with skin before a light pulse is generated to avoid the light pulse being directed into the eyes of the user or subject.

In some embodiments the parameter can be capacitance, and so the skin contact sensors 14, 16 can measure capacitance via a respective pair of electrical contacts or electrodes on the surface of the head portion 6, with the measured capacitance being indicative of whether there is skin contact. In alternative embodiments, the parameter can be an intensity or level of light, and so the skin contact sensors 14, 16 can be light sensors that measure an intensity or level of light incident on the light sensor, with the measured intensity or level being indicative of whether there is skin contact (e.g. less/no light could indicate skin contact as the skin obscures the light sensors 14, 16, and vice versa). In other alternative embodiments, the parameter can be a measure of contact pressure, and so the skin contact sensors 14, 16 can measure contact pressure via respective pressure sensors or mechanical switches, with the measured contact pressure being indicative of whether there is skin contact.

The illustrated treatment device 2 may include an optional skin tone sensor 18 positioned on or in the head portion 6 that can be used to determine a skin tone of the skin that the head portion 6 is in contact with. The skin tone sensor 18 can measure a parameter that is indicative of the skin tone of the skin, and generate a measurement signal that comprises a time-series of measurements of the parameter. The measurement signal can be processed to determine the skin tone of the skin that the head portion 6 is in contact with. Typically a skin tone sensor is used in a treatment device 2, particularly a photoepilator, to make sure that the light pulse has an intensity that is appropriate for the type of skin being treated, or even to prevent a light pulse being generated if the skin type is unsuitable for light pulses (e.g. darker skin which has a much higher melanin content).

In some embodiments the optional skin tone sensor 18 can be a light sensor and the parameter measured by the light sensor can be an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

The illustrated treatment device 2 also includes a user control 20 that can be operated by the user to activate the treatment device 2 so that the head portion 6 performs the required treatment operation on the body of the subject (e.g. the generation of one or more light pulses by the one or more light source(s) 12). The user control 20 may be in the form of a switch, a button, a touch pad, etc.

As noted above, one or more sensors may be used to provide information on positions (including orientations) and/or movements of the treatment device over time so that suitable feedback can be determined, and in some embodiments the one or more sensors can be part of or be in the treatment device 2. In the case of the sensor(s) being one or more movement sensors, such as an accelerometer, gyroscope, etc., the one or more movement sensors can be part of, e.g. inside, the treatment device 2 (and as such are not shown in Fig. 1). In embodiments where the one or more sensors includes an imaging unit (e.g. a camera), the imaging unit may be on or in the treatment device 2. As shown in Fig. 1, an imaging unit 22 may be arranged on the housing 4 of the treatment device 2 so that it is able to obtain images or a video sequence of skin or a body part that is in front of the treatment device 2. That is, the imaging unit 22 is arranged on the housing 4 of the treatment device 2 so that it obtains images or a video sequence from a direction that is generally parallel to the direction in which light pulses are emitted by the light source(s) 12 through the aperture 10. In some implementations, the imaging unit 22 is arranged on the housing 4 such that, when the head portion 6 is placed into contact with the subject so that a light pulse can be applied to the part of the skin visible to the light source(s) 12 through the aperture 10, the imaging unit 22 is not able to view the part of the skin in contact with the aperture 10 and head end 6. In embodiments where the imaging unit 22 is arranged on or in the housing 4 of the treatment device 2, the imaging unit 22 is in a fixed arrangement with respect to the rest of the treatment device 2.

A system according to the present invention generally comprises a treatment device, e.g. as shown in Fig. 1, a first sensor for outputting a first measurement signal comprising information about positions and/or movements of the treatment device over time and an apparatus, as described in more detail below, for use with the treatment device, in particular for controlling the treatment device.

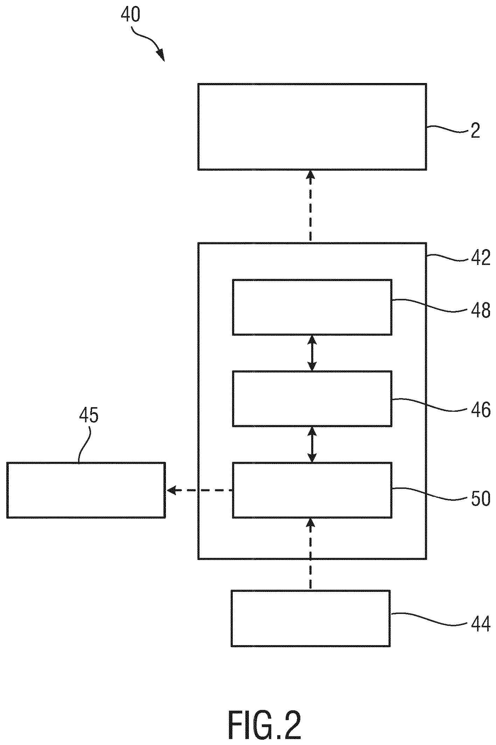

Fig. 2 is a schematic diagram of an exemplary system 40 according to the present invention comprising a treatment device 2 and an apparatus 42 for controlling the treatment device 2. The system 40 also comprises one or more sensors 44 for providing a measurement signal comprising information about positions and/or movements of the treatment device 2 over time. Further, the system may optionally comprise a feedback unit 45 for providing feedback to the user and/or subject on areas of skin that have/have not been treated with a light pulse. As noted above, in some embodiments the apparatus 42 can be a separate device to the treatment device 2, and thus the apparatus 42 may be in the form of an electronic device, such as a smart phone, smart watch, tablet, personal digital assistant (PDA), laptop, desktop computer, remote server, smart mirror, etc. **In** other embodiments, the apparatus 42, and particularly the functionality according to the invention provided by the apparatus 42, may be part of the treatment device 2.

As noted above, the one or more sensors 44 can be one or more movement sensors 44, such as an accelerometer, a gyroscope, an air pressure sensor (that can be used for measuring altitude changes, a magnetometer, etc., that measure the movements of the treatment device 2 and output a measurement signal representing those movements. The one or more movement sensors 44 may be part of or in the treatment device 2. In the case of the one or more movement sensors 44 comprising an accelerometer, the accelerometer 44 can generate a measurement signal that contains a plurality of acceleration measurement samples representing the movements of the treatment device 2 at a plurality of time instants. The accelerometer 44 may be an accelerometer that measures accelerations in three dimensions, and the measurement signal generated by the accelerometer 44 can include respective measurement signals representing the accelerations in each of the three dimensions. For example, the accelerometer 44 can output respective measurement signals for each of an x-axis, y-axis and z-axis of a Cartesian coordinate system.

The one or more sensors 44 can also include or alternatively be an imaging unit 44 (e.g. a camera or an event camera) that obtains a plurality of images or a video sequence. An event camera is an imaging sensor in which each pixel of the sensor independently indicates changes in brightness as they occur. The plurality of images (including images formed from brightness indications from an event camera) or video sequence are output as a measurement signal, and the measurement signal (images/video sequence) can be processed using imaging processing techniques to identify movements of the treatment device 2. **In** some embodiments, the imaging unit 44 is attached to or part of the treatment device 2, in which case the images/video sequence can be processed to extract the movements of the treatment device 2 based on movements of objects (e.g. body parts or skin) visible in the images/video sequence. **In** other embodiments the imaging unit 44 can be separate from the treatment device 2, e.g. an imaging unit 4 in the apparatus 44, or in a separate device such as a smart phone, smart watch, tablet, personal digital assistant (PDA), laptop, desktop computer, smart mirror, etc. **In** this case, the imaging unit 44 may be able to observe both the treatment device 2 and the body part from a distance. An imaging unit 44 may include any suitable components for capturing an image, for example a charge-coupled device (CCD) and one or more lenses and/or mirrors. **In** some embodiments, the imaging unit 44 is a camera, such as a digital camera (e.g. thermal, RGB or monochrome camera), or an event camera. As noted above, in some embodiments, the one or more sensors 44 can comprise one or more movement sensors and an imaging unit.

The apparatus 42 comprises a processing unit 46 that generally controls the operation of the apparatus 42 and enables the apparatus 42 to perform the method and techniques described herein. The processing unit 44 may further be configured to receive a measurement signal from a sensor 44, process the measurement signal to determine the feedback to be provided about the treatment operation, and provide a feedback control signal to the feedback unit 45. Thus the processing unit 46 can be configured to receive the measurement signal from the one or more sensors 44 either directly in embodiments where the sensor(s) 44 are part of the apparatus 42, or via another component in embodiments where the sensor(s) 44 are separate from the apparatus 42. In either case, the processing unit 46 can include or comprise one or more input ports or wires for receiving the measurement signal. The processing unit 46 can also include or comprise one or more output ports or wires for outputting the feedback control signal.

The processing unit 46 can be implemented in numerous ways, with software and/or hardware, to perform the various functions described herein. The processing unit 46 may comprise one or more microprocessors or digital signal processors (DSPs) that may be programmed using software or computer program code to perform the required functions and/or to control components of the processing unit 46 to effect the required functions. The processing unit 46 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI') hardware accelerators (i.e. a processor(s) or other hardware specifically designed for AI applications that can be used alongside a main processor) and/or hardware specifically designed for simultaneous localisation and mapping (SLAM) techniques.

The processing unit 46 may optionally comprise or be associated with a memory unit 48. The memory unit 48 can store data, information and/or signals (including image(s)) for use by the processing unit 46 in controlling the operation of the apparatus 42 and/or in executing or performing the methods described herein. In some implementations the memory unit 48 stores computer-readable code that can be executed by the processing unit 46 so that the processing unit 46 performs one or more functions, including the methods described herein. In particular embodiments, the program code can be in the form of an application for a smart phone, tablet, laptop, computer or server. The memory unit 48 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment shown in Fig. 2, as the apparatus 42 is shown as being separate from the sensor(s) 44, the apparatus 42 may include interface circuitry 50 to enable the apparatus 42 to receive the measurement signal(s) from the sensor(s) 44. The interface circuitry 50 in the apparatus 42 enables a data connection to and/or data exchange with other devices, including any one or more of the sensor(s) 44, the treatment device 2, servers and databases. The connection to the sensor(s) 44 (or any electronic device, such as treatment device 2) may be direct or indirect (e.g. via the Internet), and thus the interface circuitry 50 can enable a connection between the apparatus 42 and a network, or directly between the apparatus 42 and another device (such as sensor(s) 44 and/or treatment device 2), via any desirable wired or wireless communication protocol. For example, the interface circuitry 50 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 50 (and thus apparatus 42) may include one or more suitable antennas for transmitting/receiving over a transmission medium (e.g. the air). Alternatively, in the case of a wireless connection, the interface circuitry 50 may include means (e.g. a connector or plug) to enable the interface circuitry 50 to be connected to one or more suitable antennas external to the apparatus 42 for transmitting/receiving over a transmission medium (e.g. the air). The interface circuitry 50 is connected to the processing unit 46.

Although not shown in Fig. 2, the apparatus 42 may comprise one or more user interface components that includes one or more components that enables a user of apparatus 42 to input information, data and/or commands into the apparatus 42, and/or enables the apparatus 42 to output information or data to the user of the apparatus 42. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

The processing unit 46 may be configured to generate a guidance signal for the feedback unit 45, wherein the guidance signal is configured to cause the feedback unit to generate guidance indicating into which direction and/or at which treatment area the treatment device shall be moved. The feedback unit 45 may further generate feedback whether the current treatment area corresponds, or substantially corresponds, to a previous treatment area. The feedback may generally be any of, or any combination of, visual, audible and tactile. Thus the feedback unit 45 may comprise any one or more output component(s), including but not limited to a display unit (such as a display screen, a display screen/transparent panel used for providing an augmented reality (AR) display or a projector, such as a pico-projector or micro-electromechanical system (MEMS) pico-projector), one or more lights or light elements, one or more loudspeakers, and one or more vibrating elements. **In** a preferred embodiment, the feedback unit 45 is a display unit and the feedback is a graphical display comprising graphical representations of instructions and/or a computed map of treatment areas and/or previous treatment areas and the current treatment area and/or a pattern or line. For instance, a visual guidance (lines, arrows, text, etc.) may be displayed on the skin, or areas of skin that have previously been treated with a light pulse and an area that would be treated with a light pulse according to the current location of the treatment device 2 may be displayed.

In some embodiments, the feedback unit 45 is attached to or part of the treatment device 2. In other embodiments, the feedback unit 45 is part of the apparatus 42. In these embodiments the feedback unit 45 may be part of, or make use of, any of the output components of user interface components of the apparatus 42. In further embodiments, the feedback unit 45 can be separate from the treatment device 2 and the apparatus 42, for example in a separate device such as a smart phone, smart watch, tablet, personal digital assistant (PDA), laptop, desktop computer, smart mirror, etc.

It will be appreciated that a practical implementation of an apparatus 42 may include additional components to those shown in Fig. 2. For example the apparatus 42 may also include a power supply, such as a battery, or components for enabling the apparatus 42 to be connected to a mains power supply.

In a typical light-based treatment the user of the treatment device 2 needs to manually position the treatment device 2 on the skin. A light pulse is either triggered manually or automatically. However, there are little or no user-perceptible changes to the skin or hairs on applying a light pulse or shortly after applying a light pulse, so it is difficult for a user to achieve complete coverage of a body part and/or avoid over-treating certain areas of the body part by repeating a light pulse on an area of skin that has already been treated.

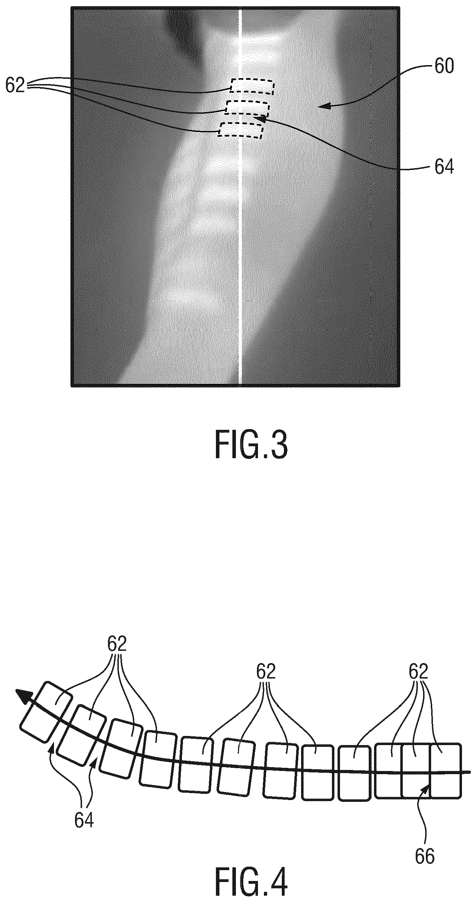

To illustrate a typical treatment coverage, Fig. 3 shows an infra-red image of an arm 60 after a number of light pulses have been applied. This infra-red image was obtained using a thermal camera. It will be appreciated that this image is included for ease of illustrating the problems addressed by the disclosed techniques, and the treatment device 2, apparatus 42 and system 40 do not include a thermal (infra-red) camera. In the image in Fig. 3, the brightness of a part of the image is dependent on the temperature of that part, with brighter (whiter) parts corresponding to higher temperatures and darker (blacker) parts corresponding to lower temperatures. It can be seen that there are a number of bright areas 62 (only some of which are labelled in Fig. 3) on the arm 60, which correspond to areas of higher temperature. These areas (also called treated treatment areas) have a higher temperature due to a light pulse having recently been applied by a treatment device 2. A light pulse will cause a small (temporary) increase in the temperature of the part of the skin that the light pulse was applied to. It can be seen in Fig. 3 that many parts of the arm 60 have not recently been treated by a light pulse, and specifically there are gaps (labelled 64) between the bright areas 62. However, there is unlikely to be a visible change to the skin caused by the light pulses, so the user of the treatment device 2 will not be able to see which parts of the arm 60 have had light pulses applied.

Fig. 4 is an illustration of a typical treatment of part of a leg in a gliding motion, where the treatment device is moved slowly at the beginning and faster at the end. Again, there may be gaps 64 between treated treatment areas 62 at the beginning and over-treated areas 66 due to the overlapping of treated treatment areas 62 at the end. furthermore, the portion of the skin (i.e. the strip of the treated treatment areas) covered by the gliding motion may not be straight but curved.

Thus, as illustrated in Figs. 3 and 4. with the conventional treatment it is generally not known to the user if there are any untreated gaps between treated treatment areas, how large (in movement direction of the treatment device) a gap between adjacent treated treatment areas is and if there adjacent treated treatment areas overlap. This illustrates the need for controlling the treatment, in particular the emission of light pulses (also called "flashing") during the user's movement of the treatment device to avoid under-treatment and over-treatment.

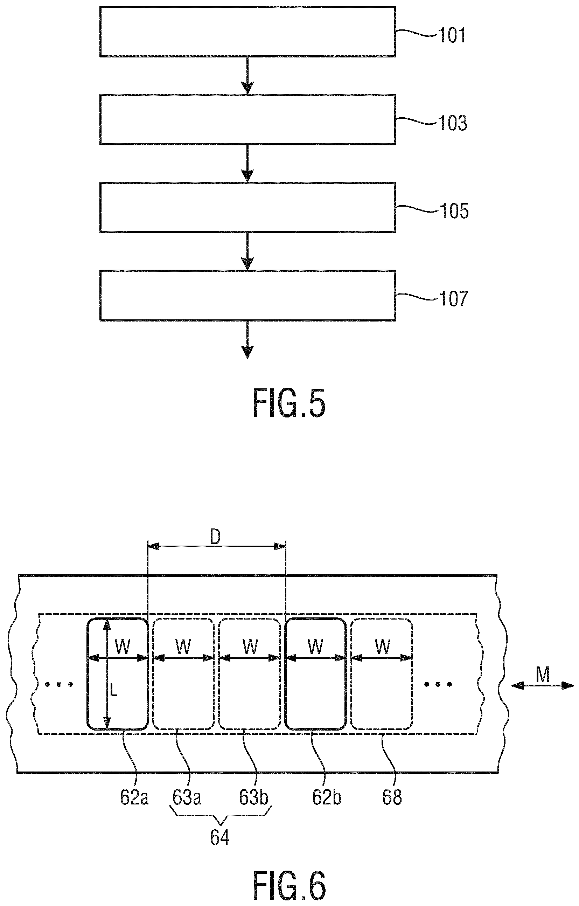

Fig. 5 shows a flow chart of an exemplary embodiment of a non-therapeutic photo-epilation method 100 according to a first aspect of the present invention. One or more of the steps of the method can be performed by the processing unit 46 in the apparatus 42. The processing unit 46 may perform the one or more steps in response to executing computer program code, that can be stored on a computer readable medium, such as, for example, the memory unit 48. Fig. 6 shows a schematic drawing used for illustrating the steps of the method 100.

In a first step 101 a position of a current treatment area 62b is estimated. The current treatment area 62b corresponds to an area of skin that the treatment device would apply a light pulse to while at the current treatment position. In a second step 103 the distance D, in movement direction M of the treatment device, of the current treatment area 62b from a previous treatment area 62a is determined. The previous treatment area corresponds to an area of skin that the treatment device applied a previous light pulse to while at a previous treatment position. In a third step 105 it is determined if the distance D is zero or an integer multiple of the width W of the treatment area in movement direction M (also called stroke direction) of the treatment device. In a fourth step 107 a pulse control signal is generated for controlling the treatment device to generate and apply a light pulse to the current treatment area 62b of the skin if the distance D has been determined to be zero or an integer multiple of the width W of the treatment area.

Thus, as illustrated in Fig. 6, the treatment device is currently at the treatment position at which the treatment area 62b will be flashed since the distance of the treatment area 62b from the previously treated treatment area 62b is two (= integer) times the width W of the treatment areas. Thus, there is a gap 64 in between the treatment areas 62a and 62b. The size of the gap 64 is, however, not of arbitrary width, but the width of the gap 64 is two times the width W of a treatment area. Thus, the gap covers two untreated treatment areas 63a, 63b, which have not been flashed while the treatment device has been moved over them, but will be flashed in subsequent stroke(s) of the portion 68 of skin (also called strip herein) to be treated in this example.

The method 100 illustrated in Fig. 5 may be continuously or regularly carried out during movement of the treatment device. Even if the speed of movement is changed during the movement it is ensured that the gap 64 in between two adjacent treated treatment areas will have a width of either zero or an integer multiple of the width W of a treatment area. If after a first strip one or more further strips of skin shall be treated, a lateral movement of the treatment device may be instructed, e.g. via a guidance signal, to laterally display the treatment device by a lateral step corresponding to the length L of a treatment area in lateral direction (perpendicular to the movement direction M so that a second strip is arranged adjacent to a first strip with substantially no gap in between the strips.

The size of a treatment area will depend on the size of the aperture 10 in the treatment device 2. In some embodiments the size of the aperture 10 is fixed or predetermined, and the processing unit 46 can make use of information on the size of the aperture 10 (e.g. the dimensions of the aperture 10), and optionally other information that may affect the size of the area of skin that the light pulse is applied to, such as relative positions of the aperture 10 and light source(s) 12, to determine the size of the treatment area. Alternatively, the processing unit 46 can determine the size of the aperture 10 by analyzing an image of the aperture 10 (e.g. if, before use, an image of the aperture 10 is obtained using an imaging unit).

As noted above, in some embodiments the treatment device 2 may have a plurality of different attachments that can be used for treating different parts of the body. These attachments may have different sized apertures 10 to change the size of the area of skin treated with each light pulse. In this case, when determining the size of the previous treatment area, the processing unit 46 can make use of information on the size of the aperture 10 (e.g. the dimensions of the aperture 10), and optionally other information such as relative positions of the aperture 10 and light source(s) 12), that was used when that light pulse was applied.

In some embodiments, the attachment that is in use on the treatment device 2 can be detected by the apparatus 42, for example by processing one or more images or a video sequence of the treatment device 2 to identify the attachment on the treatment device 2. In one example, before use of the treatment device 2, an image of the aperture 10 is obtained an imaging unit that is separate from the treatment device 2, and the processing unit 46 can determine the size of the aperture 10 by analyzing the image of the aperture 10. The size of the aperture 10 can be determined by extracting the dimensions of the window/aperture 10 using processing techniques such as edge detections and (rectangle) shape fitting.

Alternatively, the apparatus 42 can receive a signal indicating the attachment that is attached to the treatment device 2. The treatment device 2 can provide the signal in response to detecting the type of attachment that is attached to the treatment device 2. The treatment device 2 can detect the type of attachment in a number of different ways, e.g. automatically based on signals exchanged between the body of the treatment device 2 and the attachment, or based on a user setting input to the treatment device 2 indicating the type of attachment attached or the type of body part to be treated. As another option, different attachments may have apertures 10 with different colors, and the apparatus 42 can identify the attachment that is in use by observing the color of the light pulse when it is applied to the body part. Once the attachment is identified or otherwise known, information on the size of the aperture 10 associated with the identified attachment is obtained, e.g. from memory unit 48, and used to determine the size of the treatment area.

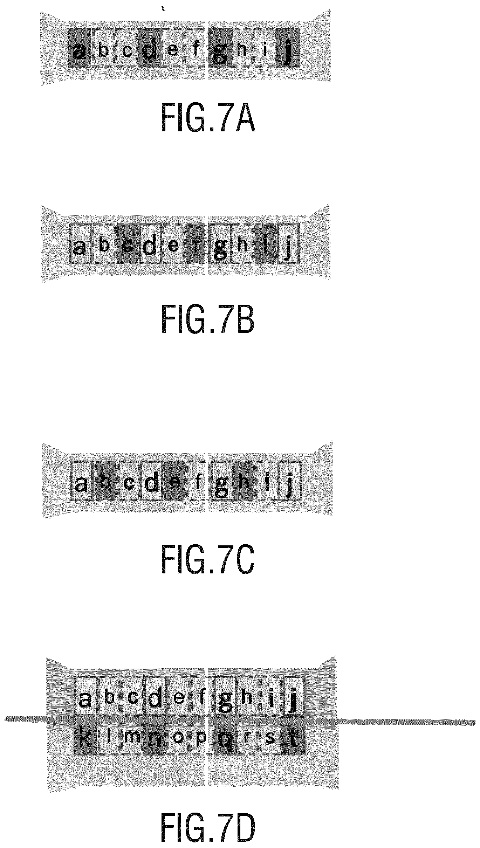

Fig. 7 shows diagrams illustrating the different strokes for the strip filling concept (i.e., the sequence in which treatment areas of a strip are flashed) shown in Fig. 6. According to Fig. 7A in a first stroke (e.g. from left to right) every third treatment area (indicated as a, d, g and j) is flashed, leaving two untreated treatment areas (indicated as b, c; e, f; h, i) in between treated treatment areas. In a second stroke (e.g. from right to left), illustrated in Fig. 7B, every third untreated treatment area (I, f, c), starting with untreated treatment area i, is flashed. In a third stroke (again from left to right), illustrated in Fig. 7C, the remaining untreated treatment areas (b, e, h) are flashed. Preferably, the user is guided with any useful indications or feedback, e.g. light paths or arrows projected onto the skin, to follow the same track and move the treatment device into the right direction.

Fig. 7D shows a fourth stroke (e.g. from right to left) used for flashing another (adjacent strip), to which the user has laterally moved the treatment device, e.g. following a light signal or projection on the skin). In this stroke the untreated treatment areas t, q, n and k are flashed as first treatment areas of the second strip. In two subsequent strokes the remaining untreated treatment areas of the second strip (in the same manner as the first strip) will be flashed.

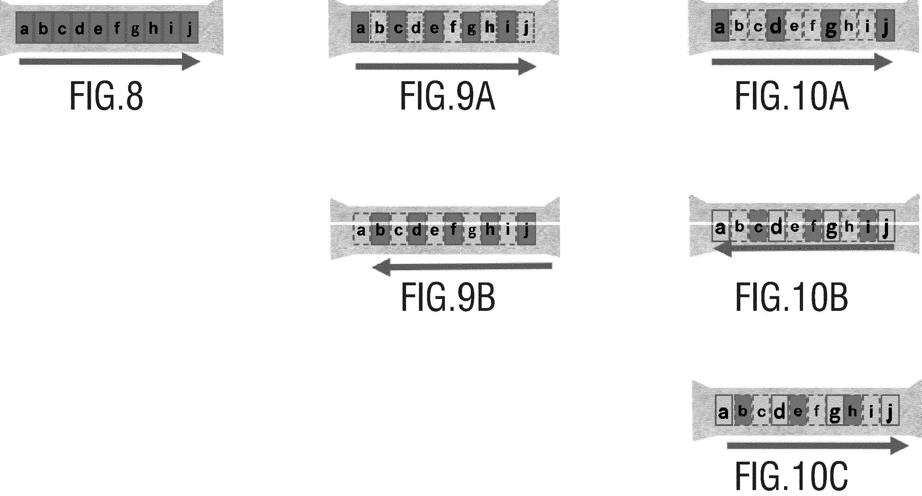

Fig. 8 shows a diagram illustrating the filling of a strip when the user applies a slow movement speed during treatment. In such a mode, every treatment area can be flashed during motion of the treatment device, i.e. the current treatment area (e.g. treatment area c) is flashed if the distance of if from the previous treated treatment area (in this example treatment area b) is zero so that the current treatment area is directly adjacent to the previous treated treatment area with (substantially) no gap in between. Completion of flashing a strip may thus be achieved in a single stroke, which may e.g. take 10 to 30 seconds (e.g. 20 seconds) at a flash rate in the range of 0.25 to 1 Hz (e.g. 0.5 Hz), depending on the length of the strip.

Fig. 9 shows diagrams illustrating the filling of a strip when the user applies a moderate movement speed during treatment. In such a mode, every second treatment area can be flashed during motion of the treatment device. In a first stroke, illustrated in Fig. 9A, the current treatment area (e.g. treatment area c) is flashed if the distance of if from the previous treated treatment area (in this example treatment area a) is one multiple of the width of the treatment area, i.e. there is a gap of one untreated treatment area between the current treatment area and the previous treated treatment area. In a second stroke, illustrated in Fig. 9B, the untreated treatment areas are then treated (i.e. the gaps left untreated in the first stroke). The second stroke is preferably applied in the opposite direction than the first stroke, which may be achieved by guiding the user accordingly, e.g. by displaying a corresponding instruction or sign on the treatment device or on the skin. Completion of flashing part of the strip in each stroke may thus be achieved in two strokes, which may each e.g. take 5 to 15 seconds (e.g. 10 seconds) at a flash rate in the range of 0.25 to 1 Hz (e.g. 0.5 Hz).

Fig. 10 shows diagrams illustrating the filling of a strip when the user applies a fast movement speed during treatment. In such a mode, every third treatment area can be flashed during motion of the treatment device. In a first stroke, illustrated in Fig. 10A, the current treatment area (e.g. treatment area d) is flashed if the distance of if from the previous treated treatment area (in this example treatment area a) is two multiples of the width of the treatment area, i.e. there is a gap of two untreated treatment areas between the current treatment area and the previous treated treatment area. In a second stroke, illustrated in Fig. 10B, every third untreated treatment area is treated (e.g. starting with treatment area i). In a third stroke, illustrated in Fig. 10C, the remaining treatment areas are treated (e.g. starting with treatment area b). The second stroke is preferably applied in the opposite direction than the first and third strokes. Completion of flashing part of the strip in each stroke may thus be achieved in three single strokes, which may each e.g. take 4 to 12 seconds (e.g. 6 or 8 seconds) at a flash rate in the range of 0.25 to 1 Hz (e.g. 0.5 Hz).

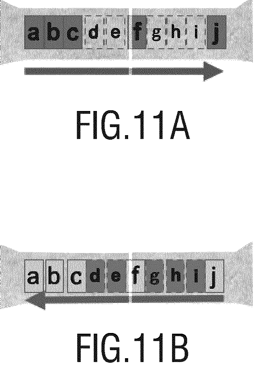

Fig. 11 shows diagrams illustrating the filling of a strip when the user applies a variable movement speed during treatment, i.e., changes the movement speed during treatment. As an example, in a first stroke, illustrated in Fig. 11A, initially a slow movement speed is applied so that the first three treatment areas a, b, c are flashed, before the user increases the speed, so that only some treatment areas f and j is flashed, leaving gaps in between. In a second stroke (preferably in opposite direction), illustrated in Fig. 11B, the user again applies a slow movement speed so that all remaining untreated treatment areas i, h, g, e and d can be flashed. Preferably, in indication is then given to the user that all treatment areas of the strip have been treated and that a lateral movement to a neighboring strip can be made if a further portion of skin shall be treated.

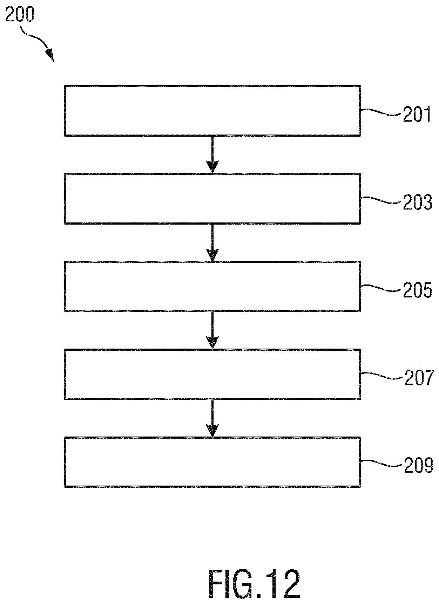

Fig. 12 shows a flow chart of an exemplary non-therapeutic photo-epilation method 200 according to the present invention for determining the current position of the treatment device.

In step 201, a first measurement signal is received from sensor 44. The first measurement signal comprises information about positions and/or movements of the treatment device 2 over time. As noted above, the first measurement signal may be a signal from a movement sensor 44, or the first measurement signal may be a signal representing a plurality of images or a video sequence. In some embodiments, step 201 may comprise receiving a first measurement signal from a first sensor 44, e.g. a movement sensor 44, and a measurement signal from another sensor 44, e.g. an imaging unit.

In step 201 the first measurement signal can be received directly from the sensor 44, for example in real-time or near real-time as the measurement signal is generated by the sensor 44. Alternatively, the first measurement signal may have been previously generated by the sensor 44 and stored for subsequent analysis, for example in the memory unit 48, in a memory unit associated with the treatment device 2 or sensor 44, or in a remote database, in which case step 201 can comprise the processing unit 46 obtaining or retrieving the first measurement signal from the storage location (e.g. from memory unit 48, etc.). In these alternative embodiments, to provide the feedback to the user at a suitable time to enable the user to complete the treatment operation the first measurement signal should be obtained or retrieved shortly (e.g. no longer than a few seconds) after the first measurement signal is generated.

In step 203, for a light pulse previously applied by the treatment device 2 to the body part during the treatment operation, the processing unit 46 processes the first measurement signal to estimate the position of the treatment device 2 relative to the body part when this light pulse was generated or applied. This estimated position is referred to as a 'previous treatment position'.

In embodiments where the sensor 44 is one or more movement sensors, such as an accelerometer, the first measurement signal is a movement measurement signal and step 203 comprises processing the movement measurement signal to identify the movements and positions of the treatment device 2 relative to the body part. Techniques for processing measurements from movement sensors to determine movements and positions of the device in which the movement sensor(s) 44 are housed are known in the art, and will not be described further herein.

In embodiments where the sensor 44 is an imaging unit and the first measurement signal is a plurality of images or a video sequence, step 203 comprises processing the images or video sequence to identify the movements and positions of the treatment device 2 relative to the body part. In embodiments where the imaging unit 44 is attached to the treatment device 2, the processing unit 46 can process the images or video sequence to identify skin features and/or body part features in the images or video sequence, and monitor the movement of those skin features and/or body part features in the images or video sequence. The determined movement of those skin features and/or body part features in the images or video sequence corresponds to the movements of the treatment device 2 relative to the body part. The imaging unit 44 is fixed in or on the treatment device 2, and the relationship between the aperture 20 and the field of view of the imaging unit 44 will be known, which enables the processing unit 46 to determine the previous treatment position, i.e. the position of the treatment device 2 relative to the body part when a light pulse was applied. Techniques for processing the images or video sequence to identify skin features and/or body part features are generally known in the art of imaging processing, although some further details are provided below.

In alternative embodiments where the imaging unit 44 is separate from the treatment device 2 and is able to observe both the treatment device 2 and the body part from a distance, in step 203 the processing unit 46 can process the images or video sequence to identify the treatment device 2 and skin features and/or body part features, and monitor their relative positions over time. Techniques for processing the images or video sequence to identify a treatment device 2 and skin features and/or body part features are generally known in the art of imaging processing, although some further details are provided below.

In embodiments where the one or more sensors 44 comprises one or more movement sensors and an imaging unit, step 203 can comprise processing a first measurement signal from one of the movement sensor(s) and the imaging unit along with a second measurement signal from the other one of the movement sensor(s) and the imaging unit to estimate the position of the treatment device 2 relative to the body part.

In some embodiments, for example in the above embodiments where the one or more sensors 44 comprises one or more movement sensors and an imaging unit, step 203 can comprise using Simultaneous Localisation And Mapping (SLAM) techniques to determine the position of the treatment device 2 relative to the body part.

In step 203, having processed the first measurement signal to estimate the movements and/or positions of the treatment device 2 relative to the body part over time, a previous treatment position is determined as the estimated position of the treatment device 2 relative to the body part at the time that the light pulse was generated or applied.

In step 205, for the light pulse evaluated in step 203 and based on the previous treatment position determined in step 203, the processing unit 46 estimates the area of skin of the body part that the light pulse was applied to. This estimated area is referred to as the 'previous treatment area' for the light pulse applied when the treatment device 2 was at the previous treatment position. The previous treatment position will generally have a known relationship with the position of the aperture 20 of the treatment device 2. This enables step 205 to determine the appropriate area of the skin on the body part that the light pulse was applied to. For example, if previous treatment positions are estimated as the geometric centre of the aperture 20, then the previous treatment area can be centred on the relevant previous treatment position. The orientation of the treatment device 2 when the light pulse was applied is used to determine the orientation of the previous treatment area on the skin. Referring to Fig. 3, a previous treatment area estimated or determined in step 205 should generally correspond to a bright area 62.

Steps 203 and 205 may be performed for a plurality of (or all) previous light pulses in the treatment operation. In preferred embodiments, steps 203 and 205 are performed when, or shortly after, a light pulse is applied to the body part or even continuously during movement of the treatment device. As such, steps 203 and 205 may be performed in response to determining that a light pulse has been applied by the treatment device 2 to the body part. The processing unit 46 may determine that a light pulse has been applied to the body part in one (or more) of a number of different ways. In embodiments where the apparatus 42 is part of the treatment device 2, the processing unit 46 can receive a signal from the light source(s) 12, or from control circuitry for the light source(s) 12, indicating that a light pulse has been generated. Alternatively, where the apparatus 42 is separate from the treatment device 2, the processing unit 46 can receive a signal from the treatment device 2 indicating that a light pulse has been generated. As another alternative, where the sensor 44 is an imaging unit, or an imaging unit is otherwise provided in the system 40, the images or video sequence from the imaging unit can be processed to identify a light pulse.

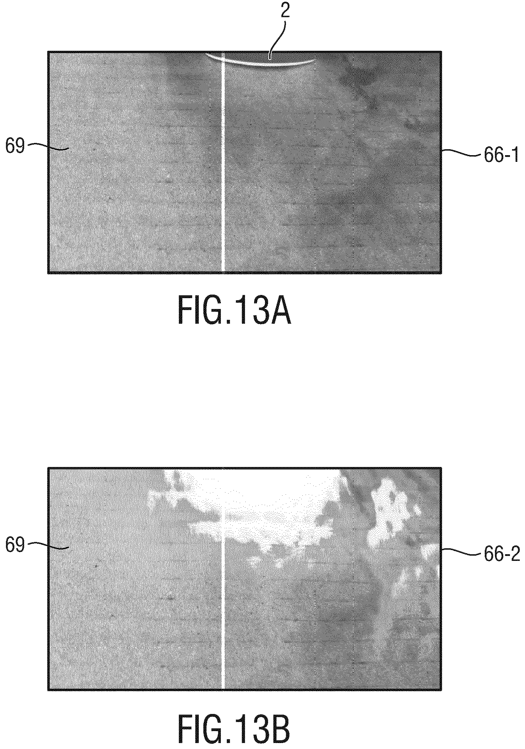

For example, Fig. 13 shows two images 66 of a body part 69 obtained by an imaging unit. In the image 66 (labelled 66-1) in Fig. 13A, part of the treatment device 2 is visible, along with the skin of the body part 69. When this image was obtained, the treatment device 2 was not applying a light pulse to the body part 69. When the image 66 (labelled 66-2) in Fig. 12B was obtained, the treatment device 2 was applying a light pulse to the body part 69, and this is visible by the brighter top part of the image 66-2. Thus, in embodiments the processing unit 46 can be configured to process images or a video sequence to identify the occurrence of a light pulse. The occurrence of a light pulse can be identified by computing an average brightness for each image (or for series of images), and comparing the average brightnesses to identify if there are any image(s) that are significantly brighter (e.g. brighter by more than a threshold amount). Alternatively the average brightness of each image or series of images can be compared to a threshold amount to determine if the brightness is consistent with a light pulse having been applied. In some embodiments, the threshold amount can be adjusted based on the environmental lighting conditions. These embodiments have the benefit that the apparatus 42 does not need to be communicatively connected to the treatment device 2, and the apparatus 42 can be used with a conventional treatment device 2.

In step 207 the processing unit 46 processes the first measurement signal to estimate a current position of the treatment device 2 relative to the body part. The processing in step 207 can be the same as used in step 203 by the processing unit 46 to estimate the position of the treatment device 2 relative to the body part when a light pulse was generated or applied.

In step 209, based on the current position of the treatment device 2 estimated in step 207, the processing unit 46 estimates the area of skin of the body part that a light pulse would be applied to in the current position of the treatment device 2. This estimated area is referred to as the 'current treatment area'. The processing in step 209 can be the same as used in step 205 by the processing unit 46 to estimate the area of skin of the body part that a previous light pulse was applied to. As in step 205, in step 209 the size of the aperture 10 can be taken into account when estimating the size of the current treatment area.

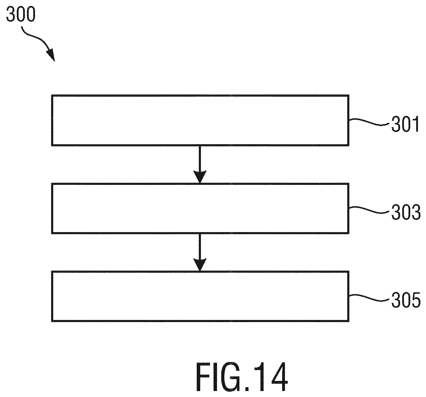

Fig. 14 shows a flow chart of an exemplary embodiment of a non-therapeutic photo-epilation method 300 according to a second aspect of the present invention. In step 301 an integer multiple of the width of the treatment area, in movement direction of the treatment device, is set based on a pre-set speed of the treatment device. In step 303 the distance, in movement direction of the treatment device, that the treatment device has moved (since the last flashing) is determined. In step 305 a pulse control signal is generated for controlling the treatment device to generate and apply a light pulse for treatment at the current position of the treatment device if the determined distance corresponds to the set integer multiple of the width of the treatment area. If the speed of movement is changed during treatment, the integer multiple may be re-calculated to correctly check the condition with respect to the distance so that - in the same manner as explained above - gaps between treated treatment areas are either zero or have a width that corresponds to an integer
multiple of a treatment area. Thus optimal treatment in minimal time can be achieved allowing the user the flexibility to use or even change the desired speed of movement of the treatment device.

As an optional step used in embodiments of the present invention, the processing unit 46 generates a feedback control signal for the feedback unit 45. The feedback control signal (or guidance signal) is generated so that it causes the feedback unit 45 to generate feedback (or guidance) indicating into which direction and/or at which treatment area the treatment device shall be moved, e.g. one or more of:
- that the treatment device shall be moved in a second stroke over the same first portion of the skin but in the opposite direction if there are untreated treatment areas between treated treatment areas in the first portion of the skin, or
- that the treatment of the first portion of the skin is finished and/or that the treatment device shall be moved in a second stroke over a second portion of the skin different from the first portion if there are no untreated treatment areas between treated treatment areas in the first portion of the skin. Other information may be indicated as well by the feedback unit.

The method may further comprise providing the generated feedback control signal to the feedback unit 45 so that the feedback is provided to the user. As noted above, the feedback unit 45 may be configured to provide any combination of visual, audible and tactile feedback to indicate whether or not the treatment device 2 is at a position where a light pulse has previously been applied. The user of the treatment device 2 would be able to use these indications to determine whether to trigger a light pulse at the current position of the treatment device 2, which can help the user to improve the coverage of the treatment operation over the skin of the subject.

As an example, the feedback control signal could cause a red light (e.g. on the treatment device 2) to be illuminated if it is determined that at the current position no light pulse shall be emitted or cause a green light (e.g. on the treatment device 2) to be illuminated if it is determined that at the current position a light pulse shall be emitted. As another example, the red/green light can be replaced with (or accompanied by) haptic feedback (e.g. vibrations of the treatment device 2). As another example, the red/green light can be replaced with (or accompanied by) audible feedback (e.g. a noise, audible message, etc.).

In a preferred embodiment, the feedback unit 45 is a display unit, such as a display screen, a transparent panel used for providing an AR display or a projector. In these embodiments, the feedback signal is a display control signal, that causes the display unit to generate a graphical display comprising graphical representations overlaid on the body part.

In embodiments where the display unit is a transparent panel used for providing an AR display, the transparent panel can be positioned in the line of sight of the user, so that it is directly between the user and the body part being treated, enabling the user to view the body part through the transparent panel. In some embodiments the transparent panel is one or both lenses in a pair of glasses. The graphical representations of the previous treatment areas and the current treatment area can be projected or displayed on the transparent panel, so that they are overlaid on the correct positions of the body part from the point of view of the user. In this way the user is presented with an AR view of the body part that can enable them to more easily see the previously treated areas on the body part.

In embodiments where feedback is to be provided during use of the treatment device 2 (e.g. as the treatment device 2 is moved over the body part and light pulse(s) are applied), steps 201-209 can be performed generally continuously. That is, in step 201 a measurement signal can be received generally continuously (e.g. according to a sampling rate of the sensor 44, or according to a transmission rate (e.g. packet rate) from the sensor 44 to the apparatus 42), and steps 203-209 can be performed generally continuously to update the feedback to be provided to the user.

In embodiments using SLAM technology where the sensor 44 is an imaging unit that is attached to the treatment device 2 and the processing unit 46 processes the images or video sequence to identify skin features and/or body part features in the images or video sequence in steps 203 and 205, the processing unit 46 can use one or more of a number of different techniques to process the images or video sequence. In some embodiments, skin features can be detected using feature detection algorithms such as Shi-Tomasi corner detection methods and/or scale-invariant feature transform (SIFT) methods. Alternatively, AI-based feature detection techniques that are optimised for skin features can be used. To estimate the movement of the treatment device 2 over time, the position of the detected skin features in the images or video sequence can be tracked over time by matching skin features between sequential images or sequential video frames. The movement of the treatment device 2 relative to the body part can be determined by calculating the homography between two sets of features of two sequential images or video frames. A homography determines the relationship between two imaging unit positions and orientations when viewing the same planar surface. Thus, as mentioned, this operation may use SLAM technology: while treating/moving, the skin area is mapped and a history of flashed skin areas is kept.

Over time, there may be a drift in the computation of the position of the current treatment position due to the accumulation of errors in the measurements and/or processing techniques. To reduce or avoid this drift, the detecting and movement tracking of skin features can be extended beyond two images or video frames so that skin features are tracked over multiple images or video frames.

Depending on the body part shown in the image(s), image processing techniques suitable for tracking position of skin features on non-planar surfaces, such as SLAM, can be used for the homography computation.

In some embodiments, the treatment device 2 may be configured to automatically trigger a light pulse. In this case the apparatus 42 can be configured to automatically trigger the treatment device 2 to generate a light pulse. In some embodiments, one or more further conditions may be applied for triggering the light pulse, such as the treatment device 2 is in contact with skin, and/or the toane of the skin the treatment device 2 is in contact with is suitable to receive a light pulse. If a light pulse can be applied at the current position of the treatment device 2, the processing unit 46 can determine an actuation signal to actuate the light source(s) 12, or that causes the treatment device 2 to actuate the light source(s) 12.

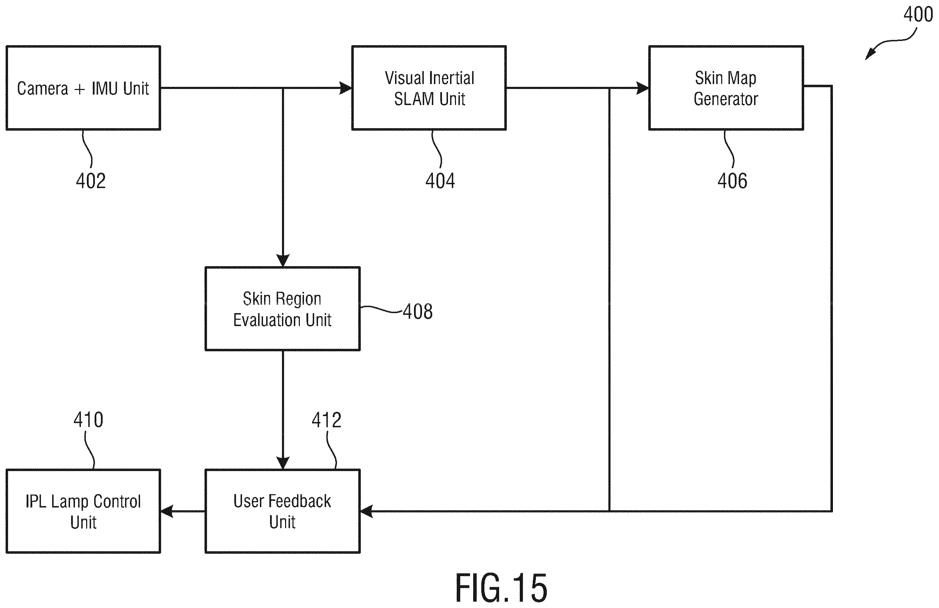

Fig. 15 shows a schematic diagram of another embodiment of a treatment device 400 according to the present invention. It comprises a camera and IMU (inertial measurement unit) 402 that captures camera images and acquires accelerometer and gyroscope values. The data are provided to a visual inertial SLAM unit 404 that computes the position and orientation of the probe (in particular the position of the treatment window). A skin map generator 406 computes a map of the treatment region for evaluation, e.g. a 3D skin map. A skin region evaluation unit 408 evaluates the camera images to identify missed spots or overtreatment. An IPL (Intense Pulse Light) lamp control unit 410 controls, e.g. using an algorithm, the flashing when the device is either directly next to a previous treated treatment area or at a distance equal to an integer number of spot size, as explained above. A user feedback unit 412 uses e.g. lights or laser projection or sound to help the user to stay 'on track'. The functions of the whole treatment device are controlled such that it is ensured that the entire desired treatment region is treated.

In an embodiment of the disclosed apparatus and method a reference skin map of the treatment region may be generated prior to a treatment session. This may be done by capturing a series of image frames of a skin portion while gliding the treatment device with camera directed onto the treatment region, recording accelerometer and gyroscope data associated to each image frame, and generating a reference skin map of the treatment region.

In an embodiment of the disclosed apparatus and method the flashing may be controlled automatically during a single- or multi-stroke treatment in a skin treatment strip. This may be done by capturing an image of skin area adjacent to the treatment area, computing the position and orientation of the treatment device based on the captured skin area image and the IMU data, defining the location and orientation of the treatment area being treated in reference to the reference skin map of the treatment region, and enabling the flashing when the location is within a pre-defined location. The pre-defined locations may be computed based on optimizing the treatment in a skin treatment strip such that minimal overlap between treatment regions occur during single- or multi-stroke treatment.

In an embodiment of the disclosed apparatus and method user guidance may be provided to a user during sliding of the treatment device during treatment along the first and subsequent skin treatment strips. This may be done by capturing image of a skin area adjacent to the treatment area, computing the position and orientation of the treatment device based on the captured skin area image and the IMU data, defining the location and orientation of the treatment area being treated in reference to the reference skin map of the treatment region, and providing visual guidance, e.g. a laser projection on skin, to a user for guidance of the sliding direction such that minimal overlap between treatment strips occurs during a treatment session.

As mentioned already, SLAM (either video based or speckle based) technology may be used to locate the treated treatment areas and untreated treatment areas. The present invention ensures that only the untreated treatment areas are flashed as described above. The strip is filled with automatic pulses even when the guiding speed is not constant. In such a scenario the following steps may be executed.

The treatment starts somewhere at the edge of the treatment region to be treated. SLAM technology maps the area adjacent to the area to be treated (both forward and backward directions) and measures the distance. A gliding motion is started by the user (generally at any speed). SLAM technology provides knowledge if the current treatment area is an integer number of widths of a treatment area away from a previous treated treatment area. When the flash lamp capacitor is charged and the current treatment area is an integer number of treatment areas away from the previous treated treatment area, the a flash will occur. In the reverse direction some or all of the untreated treatment areas are 'filled up'. This is repeated until the entire 'strip' is treated. Neither the back and forth speeds nor the speed of a single stroke do necessarily have to be the same.

To avoid treatment strip deviation between strokes, the user can be guided by a laser line during each stroke. To treat an adjacent strip without gaps between the strips (comparable to lawn mowing), the user may be guided as well.

For lateral repositioning for a second 'strip' a line (with laser, or flashing), or LEDs indicating right or left, demarcating the previous scan, may be used. This allows the user to repeat the previous scans, now laterally, adjacent to the previous scan. All the user has to do is follow the line; pulses are applied automatically.

In another embodiment, the speed guidance can be given by audio signal. Even though it is perfectly fine to treat subsequent strips at different speeds, it may be preferred by the user to do each strip in the same number of trips. A 'speed' guidance (using audio, or light) will warn if a user is about to depart from the preferred speed

In another embodiment, AI based feedback may be given. After, or even during, a treatment, tips are given about the coverage that may be reached if a slower or faster speed is used. Using AI, the patterns that were treated at which speed, indications may be given on how the user best treats at which speed. For example, many users may find it more comfortable to go at a higher speed in one direction than another. With AI, this preference could be 'learned' or noticed at least after which the sounds (or other indications) for 'speed guidance' are matching this preference.

In summary, with the present invention overtreatment and undertreatment are substantially reduced or even avoided. Further, total treatment time may be considerably reduced. The treatment can proceed at a fast speed since it is only limited by the device (charging time); no time is lost at trying to place the device at the right spot. Due to the reduced impact on the skin, irritation could potentially be reduced. Additional benefits may be increased efficiencies, reduced power consumption, optimal treatment and flexibility to use the device at any desired speed.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. An apparatus (42) configured to control a treatment device (2) configured to perform a treatment operation on a body part of a subject, wherein the treatment device is configured to apply light pulses to skin of the body part to perform the treatment operation, wherein a light pulse applied to the skin treats a treatment area of the skin, the treatment area having a known or estimated width in a movement direction of the treatment device, the apparatus (42) comprising a processing unit (46) configured to:
- estimate a position of a current treatment area corresponding to an area of skin that the treatment device would apply a light pulse to while at a current treatment position;
- determine a distance, in the movement direction of the treatment device, of the current treatment area from a previous treatment area corresponding to an area of skin that the treatment device applied a previous light pulse to while at a previous treatment position;
- determine if the distance is zero; and
- generate a pulse control signal for controlling the treatment device to generate and apply a light pulse to the current treatment area of the skin if the distance has been determined to be zero; **characterized in that** the processing unit (46) is further configured to:
- determine if the distance is an integer multiple of the width of the treatment area; and
- generate the pulse control signal for controlling the treatment device to generate and apply a light pulse to the current treatment area of the skin if the distance has been determined to be said integer multiple of the width of the treatment area.

2. The apparatus as claimed in claim 1, wherein the processing unit (46) is configured to determine the integer multiple based on an initial movement speed of the treatment device, in particular a measured or pre-set movement speed of the treatment device, and the known or estimated width of the treatment area.

3. The apparatus as claimed in claim 2, wherein the processing unit (46) is further configured to determine a number of strokes needed to treat a first portion of skin.

4. The apparatus as claimed in claim 2 or 3, wherein the processing unit (46) is further configured to adjust the integer multiple and/or the number of strokes upon determining that a current movement speed of the treatment device is different from the initial movement speed.

5. The apparatus as claimed in any one of the preceding claims, wherein the processing unit (46) is configured to:
- determine if the current treatment area overlaps with the previous treatment area, in particular by determining if a light pulse has been previously applied to the current treatment area; and
- generate the pulse control signal if the distance has been determined to be zero or an integer multiple of the width of the treatment area and if the current treatment area has been determined not to overlap with the previous treatment area.

6. The apparatus as claimed in any one of the preceding claims, wherein the processing unit (46) is configured to:
- determine if the treatment device is ready to apply a light pulse, in particular if a light source of the treatment device has sufficient energy to apply a light pulse; and
- generate the pulse control signal if the distance has been determined to be zero or an integer multiple of the width of the treatment area and if the treatment device has been determined to be ready to apply a light pulse.

7. The apparatus as claimed in any one of the preceding claims, wherein the processing unit (46) is configured to generate a guidance signal for a feedback unit (45), wherein the guidance signal is configured to cause the feedback unit (45) to generate guidance indicating into which direction and/or at which treatment area the treatment device shall be moved.

8. The apparatus as claimed in claim 7, wherein the processing unit (46) is configured to:
- determine if, after a first stroke of the treatment device over a first portion of the skin, there are untreated treatment areas between treated treatment areas; and
- generate the guidance signal for the feedback unit (45), wherein the guidance signal is configured to cause the feedback unit (45) to generate guidance indicating:
- that the treatment device shall be moved in a second stroke over the same first portion of the skin but in the opposite direction if there are untreated treatment areas between treated treatment areas in the first portion of the skin, or
- that the treatment of the first portion of the skin is finished and/or that the treatment device shall be moved in a second stroke over a second portion of the skin different from the first portion if there are no untreated treatment areas between treated treatment areas in the first portion of the skin.

9. The apparatus as claimed in claim 8, wherein the guidance signal is configured to cause the feedback unit (45) to generate guidance indicating that the treatment device shall be moved in the second stroke over a second portion of the skin that is parallel and adjacent to the first portion of the skin if there are no untreated treatment areas between treated treatment areas in the first portion of the skin.

10. The apparatus as claimed in any one of claims 7 to 9, wherein the processing unit (46) is configured to:
- compute a map indicating treated treatment areas to which a light pulse has been previously applied and untreated treatment areas to which a light pulse has not been previously applied; and
- generate the guidance signal for the feedback unit (45) based on the computed map.

11. The apparatus as claimed in any one of the preceding claims, wherein the processing unit (46) is configured to:
receive a first measurement signal from a sensor (44), the first measurement signal comprising information about positions and/or movements of the treatment device over time;
for a light pulse previously applied by the treatment device to the body part during the treatment operation, process the first measurement signal to estimate the previous treatment position as a position of the treatment device relative to the body part when the light pulse was generated;
for the previously applied light pulse and based on the estimated previous treatment position, estimate the position of the previous treatment area;
process the first measurement signal to estimate the current position of the treatment device relative to the body part; and
based on the estimated current position of the treatment device, estimate the position of the current treatment area.

12. An apparatus (42) configured to control a treatment device (2) configured to perform a treatment operation on a body part of a subject, wherein the treatment device is configured to apply light pulses to skin of the body part to perform the treatment operation, wherein a light pulse applied to the skin treats a treatment area of the skin, the treatment area having a known or estimated width in a movement direction of the treatment device, the apparatus (42) comprising a processing unit (46) configured to:
- determine a distance, in the movement direction of the treatment device, that the treatment device has moved; and
- generate, based on a pre-set speed of the treatment device, a pulse control signal for controlling the treatment device to generate and apply a light pulse if the determined distance corresponds to the width of the treatment area;
**characterized in that** the processing unit (46) is further configured to:
- set an integer multiple of the width of the treatment area based on the pre-set speed of the treatment device, wherein said integer multiple is at least two; and
- generate the pulse control signal for controlling the treatment device to generate and apply a light pulse if the determined distance corresponds to the set integer multiple of the width of the treatment area.

13. A system (40), comprising:
a treatment device (2) comprising one or more light sources for generating light pulses for application to skin of the body to perform a treatment operation;
a sensor (44) for outputting a first measurement signal comprising information about positions and/or movements of the treatment device over time; and
an apparatus (42) as claimed in any one of the preceding claims.

14. A computer-implemented non-therapeutic photo-epilation method of controlling a treatment device (2) configured to perform a treatment operation on a body part of a subject, wherein the treatment device is configured to apply light pulses to skin of the body part to perform the treatment operation, wherein a light pulse applied to the skin treats a treatment area of the skin, the treatment area having a known or estimated width in a movement direction of the treatment device, the method comprising:
- estimating a position of a current treatment area corresponding to an area of skin that the treatment device would apply a light pulse to while at a current treatment position;
- determining a distance, in the movement direction of the treatment device, of the current treatment area from a previous treatment area corresponding to an area of skin that the treatment device applied a previous light pulse to while at a previous treatment position;
- determining if the distance is zero; and
- generating a pulse control signal for controlling the treatment device to generate and apply a light pulse to the current treatment area of the skin if the distance has been determined to be zero; **characterized in that** the method further comprises:
- determining if the distance is an integer multiple of the width of the treatment area; and
- generating the pulse control signal for controlling the treatment device to generate and apply a light pulse to the current treatment area of the skin if the distance has been determined to be said integer multiple of the width of the treatment area.

15. Computer program comprising program code means for causing a computer to carry out the steps of the method as claimed in claim 14 when said computer program is carried out on the computer.

## Patentansprüche

1. Einrichtung (42), die zum Steuern einer Behandlungsvorrichtung (2) konfiguriert ist, die zum Durchführen eines Behandlungsvorgangs an einem Körperteil eines Patienten konfiguriert ist, wobei die Behandlungsvorrichtung zum Anwenden von Lichtimpulsen auf Haut des Körperteils konfiguriert ist, um den Behandlungsvorgang durchzuführen, wobei ein auf die Haut angewendeter Lichtimpuls einen Behandlungsbereich der Haut behandelt, wobei der Behandlungsbereich eine bekannte oder geschätzte Breite in einer Bewegungsrichtung der Behandlungsvorrichtung aufweist, wobei die Einrichtung (42) eine Verarbeitungseinheit (46) umfasst, die für Folgendes konfiguriert ist:
- Schätzen einer Position eines aktuellen Behandlungsbereichs, der einem Hautbereich entspricht, auf den die Behandlungsvorrichtung in der aktuellen Behandlungsposition einen Lichtimpuls anwenden würde;
- Bestimmen einer Distanz, in der Bewegungsrichtung der Behandlungsvorrichtung, des aktuellen Behandlungsbereichs von einem vorherigen Behandlungsbereich, der einem Hautbereich entspricht, auf den die Behandlungsvorrichtung in einer vorherigen Behandlungsposition einen vorherigen Lichtimpuls angewendet hat.
- Bestimmen, ob die Distanz null ist; und
- Erzeugen eines Impulssteuersignals zum Steuern der Behandlungsvorrichtung, um einen Lichtimpuls zu erzeugen und auf den aktuellen Behandlungsbereich der Haut anzuwenden, wenn die Distanz als null bestimmt wurde; **dadurch gekennzeichnet, dass** die Verarbeitungseinheit (46) weiter für Folgendes konfiguriert ist:
- Bestimmen, ob die Distanz ein ganzzahliges Vielfaches der Breite des Behandlungsbereichs ist; und
- Erzeugen des Impulssteuersignals zum Steuern der Behandlungsvorrichtung, um einen Lichtimpuls zu erzeugen und auf den aktuellen Behandlungsbereich der Haut anzuwenden, wenn die Distanz als das ganzzahlige Vielfache der Breite des Behandlungsbereichs bestimmt wurde.

2. Einrichtung nach Anspruch 1, wobei die Verarbeitungseinheit (46) dazu konfiguriert ist, das ganzzahlige Vielfache basierend auf einer anfänglichen Bewegungsgeschwindigkeit der Behandlungsvorrichtung, insbesondere einer gemessenen oder voreingestellten Bewegungsgeschwindigkeit der Behandlungsvorrichtung, und der bekannten oder geschätzten Breite des Behandlungsbereichs zu bestimmen.

3. Einrichtung nach Anspruch 2, wobei die Verarbeitungseinheit (46) weiter dazu konfiguriert ist, eine Anzahl von Durchgängen zu bestimmen, die erforderlich sind, um einen ersten Hautabschnitt zu behandeln.

4. Einrichtung nach Anspruch 2 oder 3, wobei die Verarbeitungseinheit (46) weiter dazu konfiguriert ist, das ganzzahlige Vielfache und/oder die Anzahl von Durchgängen anzupassen, wenn bestimmt wird, dass sich eine aktuelle Bewegungsgeschwindigkeit der Behandlungsvorrichtung von der anfänglichen Bewegungsgeschwindigkeit unterscheidet.

5. Einrichtung nach einem der vorstehenden Ansprüche, wobei die Verarbeitungseinheit (46) für Folgendes konfiguriert ist:
- Bestimmen, ob sich der aktuelle Behandlungsbereich mit dem vorherigen Behandlungsbereich überlappt, insbesondere durch Bestimmen, ob zuvor ein Lichtimpuls auf den aktuellen Behandlungsbereich angewendet wurde; und
- Erzeugen des Impulssteuersignals, wenn die Distanz als null oder ein ganzzahliges Vielfaches der Breite des Behandlungsbereichs bestimmt wurde und wenn der aktuelle Behandlungsbereich bestimmt wurde, nicht mit dem vorherigen Behandlungsbereich zu überlappen.

6. Einrichtung nach einem der vorstehenden Ansprüche, wobei die Verarbeitungseinheit (46) für Folgendes konfiguriert ist:
- Bestimmen, ob die Behandlungsvorrichtung bereit ist, einen Lichtimpuls anzuwenden, insbesondere ob eine Lichtquelle der Behandlungsvorrichtung über genügend Energie verfügt, um einen Lichtimpuls anzuwenden; und
- Erzeugen des Impulssteuersignals, wenn die Distanz als null oder als ein ganzzahliges Vielfaches der Breite des Behandlungsbereichs bestimmt wurde, und wenn die Behandlungsvorrichtung bestimmt wurde, bereit zu sein, einen Lichtimpuls anzuwenden.

7. Einrichtung nach einem der vorstehenden Ansprüche, wobei die Verarbeitungseinheit (46) dazu konfiguriert ist, ein Führungssignal für eine Rückkopplungseinheit (45) zu erzeugen, wobei das Führungssignal dazu konfiguriert ist, die Rückkopplungseinheit (45) zu veranlassen, eine Führung zu erzeugen, die angibt, in welche Richtung und/oder zu welchem Behandlungsbereich die Behandlungsvorrichtung bewegt werden soll.

8. Einrichtung nach Anspruch 7, wobei die Verarbeitungseinheit (46) für Folgendes konfiguriert ist:
- Bestimmen, ob nach einem ersten Durchgang der Behandlungsvorrichtung über einen ersten Hautabschnitt unbehandelte Behandlungsbereiche zwischen den behandelten Behandlungsbereichen vorhanden sind; und
- Erzeugen des Führungssignals für die Rückkopplungseinheit (45), wobei das Führungssignal dazu konfiguriert ist, die Rückkopplungseinheit (45) zu veranlassen, eine Führung zu erzeugen, die Folgendes angibt:
- dass die Behandlungsvorrichtung in einem zweiten Durchgang über denselben ersten Hautabschnitt, jedoch in der entgegengesetzten Richtung, bewegt werden soll, wenn unbehandelte Behandlungsbereiche zwischen den behandelten Behandlungsbereichen im ersten Hautabschnitt vorhanden sind, oder
- dass die Behandlung des ersten Hautabschnitts beendet ist und/oder dass die Behandlungsvorrichtung in einem zweiten Durchgang über einen zweiten Hautabschnitt, der sich vom ersten Abschnitt unterscheidet, bewegt werden soll, wenn keine unbehandelten Behandlungsbereiche zwischen den behandelten Behandlungsbereichen im ersten Hautabschnitt vorhanden sind.

9. Einrichtung nach Anspruch 8, wobei das Führungssignal dazu konfiguriert ist, die Rückmeldungseinheit (45) zu veranlassen, eine Führung zu erzeugen, die angibt, dass die Behandlungsvorrichtung im zweiten Durchgang über einen zweiten Hautabschnitt bewegt werden soll, der parallel zum und angrenzend an den ersten Hautabschnitt ist, wenn keine unbehandelten Behandlungsbereiche zwischen den behandelten Behandlungsbereichen im ersten Hautabschnitt vorhanden sind.

10. Einrichtung nach einem der Ansprüche 7 bis 9, wobei die Verarbeitungseinheit (46) für Folgendes konfiguriert ist:
- Berechnen einer Karte, die behandelte Behandlungsbereiche, auf die zuvor ein Lichtimpuls angewendet wurde, und unbehandelte Behandlungsbereiche, auf die zuvor kein Lichtimpuls angewendet wurde, angibt; und
- Erzeugen des Führungssignals für die Rückmeldungseinheit (45) basierend auf der berechneten Karte.

11. Einrichtung nach einem der vorstehenden Ansprüche, wobei die Verarbeitungseinheit (46) für Folgendes konfiguriert ist:
Empfangen eines ersten Messsignals von einem Sensor (44), wobei das erste Messsignal Informationen über Positionen und/oder Bewegungen der Behandlungsvorrichtung im Laufe der Zeit umfasst;
Verarbeiten des ersten Messsignals für einen zuvor während des Behandlungsvorgangs von der Behandlungsvorrichtung auf den Körperteil angewendeten Lichtimpuls, um die vorherige Behandlungsposition als eine Position der Behandlungsvorrichtung in Bezug zu dem Körperteil zu schätzen, als der Lichtimpuls erzeugt wurde;
Schätzen der Position des vorherigen Behandlungsbereichs für den zuvor angewendeten Lichtimpuls und basierend auf der geschätzten vorherigen Behandlungsposition;
Verarbeiten des ersten Messsignals, um die aktuelle Position der Behandlungsvorrichtung in Bezug zu dem Körperteil zu schätzen; und
Schätzen der Position des aktuellen Behandlungsbereichs basierend auf der geschätzten aktuellen Position der Behandlungsvorrichtung.

12. Einrichtung (42), die zum Steuern einer Behandlungsvorrichtung (2) konfiguriert ist, die zum Durchführen eines Behandlungsvorgangs an einem Körperteil eines Patienten konfiguriert ist, wobei die Behandlungsvorrichtung zum Anwenden von Lichtimpulsen auf Haut des Körperteils konfiguriert ist, um den Behandlungsvorgang durchzuführen, wobei ein auf die Haut angewendeter Lichtimpuls einen Behandlungsbereich der Haut behandelt, wobei der Behandlungsbereich eine bekannte oder geschätzte Breite in einer Bewegungsrichtung der Behandlungsvorrichtung aufweist, wobei die Einrichtung (42) eine Verarbeitungseinheit (46) umfasst, die für Folgendes konfiguriert ist:
- Bestimmen einer Distanz, in der Bewegungsrichtung der Behandlungsvorrichtung, die die Behandlungsvorrichtung bewegt hat; und
- Erzeugen, basierend auf einer voreingestellten Geschwindigkeit der Behandlungsvorrichtung, eines Impulssteuersignals zum Steuern der Behandlungsvorrichtung, um einen Lichtimpuls zu erzeugen und anzuwenden, wenn die bestimmte Distanz der Breite des Behandlungsbereichs entspricht;
**dadurch gekennzeichnet, dass** die Verarbeitungseinheit (46) weiter für Folgendes konfiguriert ist:
- Einstellen eines ganzzahligen Vielfachen der Breite des Behandlungsbereichs basierend auf der voreingestellten Geschwindigkeit der Behandlungsvorrichtung, wobei das ganzzahlige Vielfache mindestens zwei beträgt; und
- Erzeugen des Impulssteuersignals zum Steuern der Behandlungsvorrichtung, um einen Lichtimpuls zu erzeugen und anzuwenden, wenn die bestimmte Distanz dem eingestellten ganzzahligen Vielfachen der Breite des Behandlungsbereichs entspricht.

13. System (40), umfassend:
eine Behandlungsvorrichtung (2), die eine oder mehrere Lichtquellen zum Erzeugen von Lichtimpulsen zur Anwendung auf Haut des Körpers umfasst, um einen Behandlungsvorgang durchzuführen;
einen Sensor (44) zum Ausgeben eines ersten Messsignals, das Informationen über Positionen und/oder Bewegungen der Behandlungsvorrichtung im Laufe der Zeit umfasst; und
eine Einrichtung (42) nach einem der vorstehenden Ansprüche.

14. Computerimplementiertes nicht-therapeutisches Photoepilationsverfahren zum Steuern einer Behandlungsvorrichtung (2), die zum Durchführen eines Behandlungsvorgangs an einem Körperteil eines Patienten konfiguriert ist, wobei die Behandlungsvorrichtung zum Anwenden von Lichtimpulsen auf Haut des Körperteils konfiguriert ist, um den Behandlungsvorgang durchzuführen, wobei ein auf die Haut angewendeter Lichtimpuls einen Behandlungsbereich der Haut behandelt, wobei der Behandlungsbereich eine bekannte oder geschätzte Breite in einer Bewegungsrichtung der Behandlungsvorrichtung aufweist, wobei das Verfahren Folgendes umfasst:
- Schätzen einer Position eines aktuellen Behandlungsbereichs, der einem Hautbereich entspricht, auf den die Behandlungsvorrichtung in der aktuellen Behandlungsposition einen Lichtimpuls anwenden würde;
- Bestimmen einer Distanz, in der Bewegungsrichtung der Behandlungsvorrichtung, des aktuellen Behandlungsbereichs von einem vorherigen Behandlungsbereich, der einem Hautbereich entspricht, auf den die Behandlungsvorrichtung in einer vorherigen Behandlungsposition einen vorherigen Lichtimpuls angewendet hat.
- Bestimmen, ob die Distanz null ist; und
- Erzeugen eines Impulssteuersignals zum Steuern der Behandlungsvorrichtung, um einen Lichtimpuls zu erzeugen und auf den aktuellen Behandlungsbereich der Haut anzuwenden, wenn die Distanz als null bestimmt wurde; **dadurch gekennzeichnet, dass** das Verfahren weiter Folgendes umfasst:
- Bestimmen, ob die Distanz ein ganzzahliges Vielfaches der Breite des Behandlungsbereichs ist; und
- Erzeugen des Impulssteuersignals zum Steuern der Behandlungsvorrichtung, um einen Lichtimpuls zu erzeugen und auf den aktuellen Behandlungsbereich der Haut anzuwenden, wenn die Distanz als das ganzzahlige Vielfache der Breite des Behandlungsbereichs bestimmt wurde.

15. Computerprogramm, das Programmcodemittel umfasst, um einen Computer zu veranlassen, die Schritte des Verfahrens nach Anspruch 14 auszuführen, wenn das Computerprogramm auf dem Computer ausgeführt wird.

## Revendications

1. Appareil (42) configuré pour commander un dispositif de traitement (2) configuré pour effectuer une opération de traitement sur une partie de corps d'un sujet, dans lequel le dispositif de traitement est configuré pour appliquer des impulsions lumineuses à une peau de la partie de corps afin d'effectuer l'opération de traitement, dans lequel une impulsion lumineuse appliquée à la peau traite une zone de traitement de la peau, la zone de traitement présentant une largeur connue ou estimée dans une direction de déplacement du dispositif de traitement, l'appareil (42) comprenant une unité de traitement (46) configurée pour :
- estimer une position d'une zone de traitement actuelle correspondant à une zone de peau sur laquelle le dispositif de traitement appliquerait une impulsion lumineuse alors qu'il se trouve dans une position de traitement actuelle ;
- déterminer une distance, dans la direction de déplacement du dispositif de traitement, de la zone de traitement actuelle par rapport à une zone de traitement précédente correspondant à une zone de peau sur laquelle le dispositif de traitement a appliqué une impulsion lumineuse précédente alors qu'il se trouvait dans une position de traitement précédente ;
- déterminer si la distance est nulle ; et
- générer un signal de commande d'impulsion pour commander le dispositif de traitement afin de générer et d'appliquer une impulsion lumineuse à la zone de traitement actuelle de la peau si la distance a été déterminée comme étant nulle ; **caractérisé en ce que** l'unité de traitement (46) est en outre configurée pour :
- déterminer si la distance est un multiple entier de la largeur de la zone de traitement ; et
- générer le signal de commande d'impulsion pour commander le dispositif de traitement afin de générer et d'appliquer une impulsion lumineuse à la zone de traitement actuelle de la peau si la distance a été déterminée comme étant ledit multiple entier de la largeur de la zone de traitement.

2. Appareil selon la revendication 1, dans lequel l'unité de traitement (46) est configurée pour déterminer le multiple entier sur la base d'une vitesse de déplacement initiale du dispositif de traitement, en particulier une vitesse de déplacement mesurée ou prédéfinie du dispositif de traitement, et de la largeur connue ou estimée de la zone de traitement.

3. Appareil selon la revendication 2, dans lequel l'unité de traitement (46) est en outre configurée pour déterminer un nombre de passages nécessaires pour traiter une première partie de la peau.

4. Appareil selon la revendication 2 ou 3, dans lequel l'unité de traitement (46) est en outre configurée pour ajuster le multiple entier et/ou le nombre de passages nécessaires à la détermination qu'une vitesse de déplacement actuelle du dispositif de traitement est différente de la vitesse de déplacement initiale.

5. Appareil selon l'une quelconque des revendications précédentes, dans lequel l'unité de traitement (46) est configurée pour :
- déterminer si la zone de traitement actuelle chevauche la zone de traitement précédente, notamment en déterminant si une impulsion lumineuse a été précédemment appliquée à la zone de traitement actuelle ; et
- générer le signal de commande d'impulsion si la distance a été déterminée comme étant nulle ou un multiple entier de la largeur de la zone de traitement et si la zone de traitement actuelle a été déterminée comme ne chevauchant pas la zone de traitement précédente.

6. Appareil selon l'une quelconque des revendications précédentes, dans lequel l'unité de traitement (46) est configurée pour :
- déterminer si le dispositif de traitement est prêt à appliquer une impulsion lumineuse, en particulier si une source de lumière du dispositif de traitement présente une énergie suffisante pour appliquer une impulsion lumineuse ; et
- générer le signal de commande d'impulsion si la distance a été déterminée comme étant nulle ou un multiple entier de la largeur de la zone de traitement et si le dispositif de traitement a été déterminé comme étant prêt à appliquer une impulsion lumineuse.

7. Appareil selon l'une quelconque des revendications précédentes, dans lequel l'unité de traitement (46) est configurée pour générer un signal de guidage destiné à une unité de rétroaction (45), dans lequel le signal de guidage est configuré pour amener l'unité de rétroaction (45) à générer un guidage indiquant la direction à suivre et/ou la zone de traitement vers laquelle le dispositif de traitement doit être déplacé.

8. Appareil selon la revendication 7, dans lequel l'unité de traitement (46) est configurée pour :
- déterminer si, après un premier passage du dispositif de traitement sur une première partie de peau, il existe des zones de traitement non traitées entre des zones de traitement traitées ; et
- générer le signal de guidage pour l'unité de rétroaction (45), dans lequel le signal de guidage est configuré pour amener l'unité de rétroaction (45) à générer un guidage indiquant :
- que le dispositif de traitement doit être déplacé lors d'un second passage sur la même première partie de peau, mais dans la direction opposée, s'il existe des zones de traitement non traitées entre des zones de traitement traitées dans la première partie de peau, ou
- que le traitement de la première partie de la peau est terminé et/ou que le dispositif de traitement doit être déplacé lors d'un second passage sur une seconde partie de la peau différente de la première partie s'il n'y a pas de zones de traitement non traitées entre des zones de traitement traitées dans la première partie de la peau.

9. Appareil selon la revendication 8, dans lequel le signal de guidage est configuré pour amener l'unité de rétroaction (45) à générer un guidage indiquant que le dispositif de traitement doit être déplacé lors du second passage sur une seconde partie de la peau qui est parallèle et adjacente à la première partie de la peau s'il n'y a pas de zones de traitement non traitées entre des zones de traitement traitées dans la première partie de la peau.

10. Appareil selon l'une quelconque des revendications 7 à 9, dans lequel l'unité de traitement (46) est configurée pour :
- calculer une carte indiquant des zones de traitement traitées auxquelles une impulsion lumineuse a été préalablement appliquée et des zones de traitement non traitées auxquelles aucune impulsion lumineuse n'a été préalablement appliquée ; et
- générer le signal de guidage pour l'unité de rétroaction (45) sur la base de la carte calculée.

11. Appareil selon l'une quelconque des revendications précédentes, dans lequel l'unité de traitement (46) est configurée pour :
recevoir un premier signal de mesure en provenance d'un capteur (44), le premier signal de mesure comprenant des informations concernant des positions et/ou des mouvements du dispositif de traitement au fil du temps ;
pour une impulsion lumineuse précédemment appliquée par le dispositif de traitement à la partie de corps pendant l'opération de traitement, traiter le premier signal de mesure pour estimer une position de traitement précédente en tant que position du dispositif de traitement par rapport à la partie de corps lorsque l'impulsion lumineuse a été générée ;
pour l'impulsion lumineuse précédemment appliquée et sur la base de la position de traitement précédente estimée, estimer la position de la zone de traitement précédente ;
traiter le premier signal de mesure pour estimer la position actuelle du dispositif de traitement par rapport à la partie de corps ; et
sur la base de la position actuelle estimée du dispositif de traitement, estimer la position de la zone de traitement actuelle.

12. Appareil (42) configuré pour commander un dispositif de traitement (2) configuré pour effectuer une opération de traitement sur une partie de corps d'un sujet, dans lequel le dispositif de traitement est configuré pour appliquer des impulsions lumineuses à une peau de la partie de corps afin d'effectuer l'opération de traitement, dans lequel une impulsion lumineuse appliquée à la peau traite une zone de traitement de la peau, la zone de traitement présentant une largeur connue ou estimée dans une direction de déplacement du dispositif de traitement, l'appareil (42) comprenant une unité de traitement (46) configurée pour :
- déterminer une distance parcourue par le dispositif de traitement dans la direction de déplacement du dispositif de traitement ; et
- générer, sur la base d'une vitesse prédéfinie du dispositif de traitement, un signal de commande d'impulsion pour commander le dispositif de traitement afin de générer et d'appliquer une impulsion lumineuse si la distance déterminée correspond à la largeur de la zone de traitement ;
**caractérisé en ce que** l'unité de traitement (46) est configurée en outre pour :
- définir un multiple entier de la largeur de la zone de traitement sur la base de la vitesse prédéfinie du dispositif de traitement, dans lequel ledit multiple entier est au moins égal à deux ; et
- générer le signal de commande d'impulsion pour commander le dispositif de traitement afin de générer et d'appliquer une impulsion lumineuse si la distance déterminée correspond au multiple entier défini de la largeur de la zone de traitement.

13. Système (40), comprenant :
un dispositif de traitement (2) comprenant une ou plusieurs sources de lumière pour générer des impulsions lumineuses destinées à être appliquées sur la peau du corps pour effectuer une opération de traitement ;
un capteur (44) pour émettre un premier signal de mesure comprenant des informations concernant des positions et/ou des mouvements du dispositif de traitement au fil du temps ; et
un appareil (42) selon l'une quelconque des revendications précédentes.

14. Procédé de photo-épilation non thérapeutique mis en œuvre par ordinateur, commandant un dispositif de traitement (2) configuré pour effectuer une opération de traitement sur une partie de corps d'un sujet, dans lequel le dispositif de traitement est configuré pour appliquer des impulsions lumineuses à la peau de la partie de corps afin d'effectuer l'opération de traitement, dans lequel une impulsion lumineuse appliquée à la peau traite une zone de traitement de la peau, la zone de traitement présentant une largeur connue ou estimée dans une direction de déplacement du dispositif de traitement, le procédé comprenant :
- l'estimation d'une position d'une zone de traitement actuelle correspondant à une zone de peau sur laquelle le dispositif de traitement appliquerait une impulsion lumineuse alors qu'il se trouve dans une position de traitement actuelle ;
- la détermination d'une distance, dans la direction de déplacement du dispositif de traitement, de la zone de traitement actuelle par rapport à une zone de traitement précédente correspondant à une zone de peau sur laquelle le dispositif de traitement a appliqué une impulsion lumineuse précédente alors qu'il se trouvait dans une position de traitement précédente ;
- la détermination si la distance est nulle ; et
- la génération d'un signal de commande d'impulsion pour commander le dispositif de traitement afin de générer et d'appliquer une impulsion lumineuse à la zone de traitement actuelle de la peau si la distance a été déterminée comme étant nulle ; **caractérisé en ce que** le procédé comprend en outre :
- la détermination si la distance est un multiple entier de la largeur de la zone de traitement ; et
- la génération du signal de commande d'impulsion pour commander le dispositif de traitement afin de générer et d'appliquer une impulsion lumineuse à la zone de traitement actuelle de la peau si la distance a été déterminée comme étant ledit multiple entier de la largeur de la zone de traitement.

15. Programme informatique comprenant des moyens de code de programme pour amener un ordinateur à exécuter les étapes du procédé selon la revendication 14 lorsque ledit programme informatique est exécuté sur l'ordinateur.
